# EUROPEAN PATENT APPLICATION

(11) **EP 1 719 544 A1**
(43) Date of publication of application: **08.11.2006**
(21) Application number: 05290371.3
(22) Date of filing: 18.02.2005
(51) Int. Cl.: A61Q 5/00, A61Q 5/06, A61K 8/49

(54) **Dye composition comprising an oxonol type methine direct dye, process for implementation and uses thereof**

(71) Applicant: L'ORÉAL, 75008 Paris (FR); FUJI PHOTO FILM CO., LTD., Minami-Ashigara-shi, Kanagawa (JP)
(72) Inventor: Plos, Grégory, 158-0097 Tokyo (JP); Hioki Takanori, Minato-ku Tokyo (JP)
(74) Representative: Dossmann, Gérard

(57) **Abstract**

The present patent application relates to a dye composition for dyeing keratin fibres, in particular human keratin fibres such as the hair, comprising, in a suitable dyeing medium, at least one methine direct dye containing at least two 6-membered rings.

The present patent application also relates to the dyeing process using this composition and to its uses, and also to a family of methine direct compounds.

## Description

The present patent application relates to a dye composition for dyeing keratin fibres, comprising at least one particular oxonol type methine direct dye in a suitable dyeing medium.

It is known practice to dye keratin fibres, and in particular human hair, with dye compositions containing oxidation dye precursors, which are generally known as oxidation bases, such as ortho- or para-phenylenediamines, ortho- or para-aminophenols, and heterocyclic compounds. These oxidation bases are colourless or weakly coloured compounds that, when combined with oxidizing products, may give rise to coloured compounds by a process of oxidative condensation. These dyes, which are insoluble in the dyeing medium, are trapped inside the hair.

It is also known that the shades obtained with these oxidation bases may be varied by combining them with couplers or coloration modifiers, the latter being chosen especially from aromatic metadiamines, meta-aminophenols, meta-diphenols and certain heterocyclic compounds such as indole compounds.

The variety of molecules used as oxidation bases and couplers allows a wide range of colours to be obtained.

By this way, a "permanent" coloration is obtained. Nevertheless, since the oxidation dyeing operation is performed in the presence of aqueous hydrogen peroxide solution and aqueous ammonia, bleaching and dissolution of the melanin occur, together with oxidation of the oxidation precursors. The hair is thus bleached at the same time that it is dyed. The dyeing is thus visible even on dark hair. However, the oxidation is reflected by degradation of the hair.

It is also known practice to dye keratin fibres by direct or semi-permanent dyeing. The process conventionally used in direct dyeing consists in applying to the keratin fibres direct dyes, which are coloured and colouring molecules that have affinity for the fibres, leaving the coloured molecules on the fibres to allow them to penetrate, by diffusion, into the hair, and then rinsing the fibres.

It is known practice, for example, to use nitrobenzene, anthraquinone, nitropyridine, azo, xanthene, acridine, azine or triarylmethane direct dyes.

This results in colorations that are particularly chromatic but, however, temporary or semi-permanent on account of the nature of the bonds between the direct dyes and the keratin fibre. These interactions cause the dyes to desorb readily from the surface and/or the core of the fibre. The colorations generally show weak dyeing power and poor fastness with respect to washing or perspiration. These direct dyes are also generally light-sensitive, since the resistance of the chromophore to photochemical attack is low, which leads to fading of the coloration of the hair over time. The light sensitivity of these dyes depends on their uniform distribution or distribution as aggregates in/on the keratin fibre.

In addition, this type of dyeing is generally not very visible on dark hair since the background of the hair masks the colour of the dye that has diffused inside.

On the other hand, since the application of the coloration takes place under mild conditions, i.e. without oxidizing agents, the hair is not damaged.

One object of the invention is to provide novel compositions for dyeing keratin fibres, in particular human keratin fibres, such as the hair, based on direct dyes that allow dark hair to be visibly coloured and without an oxidizing treatment in order to preserve the hair.

A first subject of the present patent application consists of a dye composition for dyeing keratin fibres, in particular human keratin fibres, such as the hair, comprising, in a suitable dyeing medium, at least one particular methine direct dye of general formula (I). 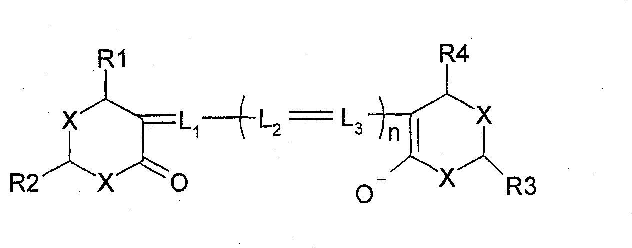 in which X represents CRR', NR", S or 0, and
R et R' represent, independently of each other, a hydrogen atom; a halogen atom such as chlorine, bromine, fluorine or iodine; a linear or branched alkyl group substituted or unsubstituted, a linear or branched alkoxy group; a hydroxyl group; a linear or branched hydroxyalkyl group; a nitro group; a sulfo group; a carboxyl group; an acetylamino group; a cyano group; a thiol group; a linear or branched alkylthiol group; a linear or branched thioalkyl group; a linear or branched alkylcarbonyl group; a hydrogenocarbonyl group; a hydroxycarbonyl group; a linear or branched alkoxycarbonyl group; an ammonio group, a mono-, di- or trialkylammonio group; an imidazolio group; a pyridinio group; a substituted or unsubstituted aromatic group; a substituted or unsubstituted heteroaromatic group; a substituted or unsubstituted oxyaromatic group; a substituted or unsubstituted oxyheteroaromatic group; or, R represents a hydrogen atom and R' represents NRaRb in which Ra and Rb, independently of each other, represent a hydrogen atom; a linear or branched C₁-C₆ alkyl group or a linear or branched C₁-C₆ hydroalkyl group;
R" represents a hydrogen atom, a linear or branched alkyl group substituted or not, a hydroxyl group; or a linear or branched hydroxyalkyl group; an alkylsulfonium group such as -(CH₂)₂₋₆SO₃Na group or an aryl group or a heteroaryl group;
R₁, R₂, R₃ and R₄ represent, independently of each other, a hydrogen atom; a halogen atom; a linear or branched alkyl group substituted or unsubstituted; a linear or branched alkoxy group; a hydroxyl group; a linear or branched hydroxyalkyl group; a nitro group; a sulfo group; a carboxyl group; a linear or branched alkylcarboxyl group, a formyl group; a linear or branched alkylcarbonyl group; a thionyl group; a group NRaRb in which Ra and Rb, independently of each other, represent a hydrogen atom; a linear or branched C₁-C₆ alkyl group or a linear or branched C₁-C₆ hydroxyalkyl group; an acetylamino group; a cyano group; a thiol group; a C₁-C₆ alkylthiol group; a linear or branched thioalkyl group, a linear or branched C₁-C₆ alkylcarbonyl group; a hydrogenocarbonyl group; a hydroxycarbonyl group; a linear or branched alkoxycarbonyl group; an ammonio group; a mono-, di- or tri(C₁-C₆)alkylammonio group; an imidazolio group; a pyridinio group; a substituted or unsubstituted aromatic group; a substituted or unsubstituted heteroaromatic group; a substituted or unsubstituted oxyaromatic group; a substituted or unsubstituted oxyheteroaromatic group; or at least one of the groups R₁, R₂, R₃ and R₄, independently of each other, forms, with the carbon atom to which it is attached, a carbonyl group;
n is an integer ranging from 0 to 20;
L1, L2 and L3 independently represent a substituted or unsubstituted methine group, and
M is the counterion necessary to balance the charge of compound of formula (I).

A second subject of the present patent application consists of a process for dyeing keratin fibres, in particular human keratin fibres such as the hair, using this composition.

Unless contrary indication, the alkyl or aryl group can be unsubstituted or substituted. The alkyl group generally comprises 1 to 18 carbon atoms, preferably 1 to 6, more preferably 1 to 4 carbon atoms.

Herein, the substituents are not particularly restricted and any substituents may be used. For example, the substituent represents a halogen atom ; an alkyl group including a cycloalkyl group and a bicycloalkyl group ; an alkenyl group including a cycloalkenyl group and a bicycloalkenyl group ; an alkynyl group ; an aryl group, a heterocyclic group ; a cyano group ; a hydroxyl group 1; a nitro group, a carboxyl group ; an alkoxyl group ; an aryloxy group ; a silyloxy group ; a heterocyclic oxy group ; an acyloxy group ; a carbamoyloxy group ; an alkoxycarbonyloxy group ; an aryloxycarbonyloxy group ; an amino group ; an ammonio group ; an acylamino group ; an aminocarbonylamino group ; an alkoxycarbonylamino group ; an aryloxycarbonylamino group ; a sulfamoylamino group ;, an alkylsulfonylamino group ; an arylsulfonylamino group ; a mercapto group ; an alkylthio group ; an arylthio group ; a heterocyclic thio group ; a sulfamoyl group ; a sulfo group ; an alkylsulfinyl group ; an arylsulfinyl group; an alkylsulfonyl group ; an arylsulfonyl group ; an acyl group ; an aryloxycarbonyl group ; an alkoxycarbonyl group ; a carbamoyl group ; an arylazo group ; a heterocyclic azo group ; an imido group ; a phosphino group ; a phosphinyl group ; a phosphinyloxy group; a phosphinylamino group ; a phospho group ; a silyl group ; a hydrazino group ; a ureido group, and other well-known substituents.

More specifically, the substituent represents a halogen atom (e.g., fluorine, chlorine, bromine, iodine), a straight chain, branched, cyclic, substituted or unsubstituted alkyl group including an alkyl group preferably an alkyl group having from 1 to 30 carbon atoms (e.g., methyl, ethyl, n-propyl, isopropyl, t-butyl, n-octyl, eicosyl, 2-chloroethyl, 2-cyanoethyl, 2-ethylhexyl), a cycloalkyl group preferably a substituted or unsubstituted cycloalkyl group having from 3 to 30 carbon atoms e.g., cyclohexyl, cyclopentyl, 4-n-dodecylcyclohexyl), a bicycloalkyl group [preferably a substituted or unsubstituted bicycloalkyl group having from 5 to 30 carbon atoms, i.e., a monovalent group obtained by eliminating one hydrogen atom from bicycloalkane having from 5 to 30 carbon atoms e.g., bicyclo[1,2,2]heptan-2-yl, bicyclo[2,2,2]octan-3-yl, and a tricyclo structure having many ring structures; the alkyl group in the substituent described below e.g., the alkyl group in an alkylthio group represents the alkyl group of such a concept and further includes an alkenyl group and an alkynyl group, a straight chain, branched, cyclic, substituted or unsubstituted alkenyl group including an alkenyl group, preferably a substituted or unsubstituted alkenyl group having from 2 to 30 carbon atoms e.g., vinyl, allyl, prenyl, geranyl, oleyl, a cycloalkenyl group preferably a substituted or unsubstituted cycloalkenyl group having from 3 to 30 carbon atoms, i.e., a monovalent group obtained by eliminating one hydrogen atom from cycloalkene having from 3 to 30 carbon atoms e.g., 2-cyclopenten-1-yl, 2-cyclohexen-1-yl, a substituted or unsubstituted bicycloalkenyl group, preferably a substituted or unsubstituted bicycloalkenyl group having from 5 to 30 carbon atoms, i.e., a monovalent group obtained by eliminating one hydrogen atom from bicycloalkene having one double bond (e.g., bicyclo[2,2,1]hepto-2-en-1-yl, bicyclo-[2,2,2]octo-2-en-4-yl))] an alkynyl group preferably a substituted or unsubstituted alkynyl group having from 2 to 30 carbon atoms e.g., ethynyl, propargyl, trimethylsilylethynyl, an aryl group [preferably a substituted or unsubstituted aryl group having from 6 to 30 carbon atoms e.g., phenyl, p-tolyl, naphthyl, m-chlorophenyl, o-hexadecanoylaminophenyl, a heterocyclic group, preferably a monovalent group obtained by eliminating one hydrogen atom from a 5- or 6-membered, substituted or unsubstituted, aromatic or nonaromatic heterocyclic compound, more preferably a 5- or 6-membered aromatic heterocyclic group having from 3 to 30 carbon atoms e.g., 2-furyl, 2-thienyl, 2-pyrimidinyl, 2-benzothiazolyl, further, a cationic heterocyclic group, e.g., 1-methyl-2-pyridinio, 1-methyl-2-quinolinio may be included, a cyano group, a hydroxyl group, a nitro group, a carboxyl group, an alkoxyl group , preferably a substituted or unsubstituted alkoxyl group having from 1 to 30 carbon atoms e.g., methoxy, ethoxy, isopropoxy, t-butoxy, n-octyloxy, 2-methoxyethoxy, an aryloxy group ,preferably a substituted or unsubstituted aryloxy group having from 6 to 30 carbon atoms e.g., phenoxy, 2-methylphenoxy, 4-t-butylphenoxy, 3-nitrophenoxy, 2-tetradecanoylaminophenoxy, a silyloxy group , preferably a silyloxy group having from 3 to 20 carbon atoms e.g., trimethylsilyloxy, t-butyldimethylsilyloxy, a heterocyclic oxy group, preferably a substituted or unsubstituted heterocyclic oxy group having from 2 to 30 carbon atoms, e.g., 1-phenyltetrazol-5-oxy, 2-tetrahydropyranyloxy, an acyloxy group, preferably a formyloxy group, a substituted or unsubstituted alkylcarbonyloxy group having from 2 to 30 carbon atoms, a substituted or unsubstituted arylcarbonyloxy group having from 6 to 30 carbon atoms e.g., formyloxy, acetyloxy, pivaloyloxy, stearoyloxy, benzoyloxy, p-methoxyphenylcarbonyloxy, a carbamoyloxy group [preferably a substituted or unsubstituted carbamoyloxy group having from 1 to 30 carbon atoms e.g., N,N-dimethylcarbamoyloxy, N,N-diethylcarbamoyloxy, morpholinocarbonyloxy, N,N-di-n-octylaminocarbonyloxy, N-n-octylcarbamoyloxy, an alkoxycarbonyloxy group, preferably a substituted or unsubstituted alkoxycarbonyloxy group having from 2 to 30 carbon atoms e.g., methoxycarbonyloxy, ethoxycarbonyloxy, t-butoxycarbonyloxy, n-octylcarbonyloxy, an aryloxycarbonyloxy group [preferably a substituted or unsubstituted aryloxycarbonyloxy group having from 7 to 30 carbon atoms e.g., phenoxycarbonyloxy, p-methoxyphenoxycarbonyloxy, p-n-hexadecyloxyphenoxycarbonyloxy, an amino group, preferably an amino group, a substituted or unsubstituted alkylamino group having from 1 to 30 carbon atoms, a substituted or unsubstituted anilino group having from 6 to 30 carbon atoms (e.g., amino, methylamino, dimethylamino, anilino, N-methylanilino, diphenylamino, an ammonio group, preferably an ammonio group, an ammonio group substituted with a substituted or unsubstituted alkyl, aryl or heterocyclic ring having from 1 to 30 carbon atoms , e.g., trimethylammonio, triethylammonio, diphenylmethylammonio, an acylamino group, preferably a formylamino group, a substituted or unsubstituted alkylcarbonylamino group having from 1 to 30 carbon atoms, a substituted or unsubstituted arylcarbonylamino group having from 6 to 30 carbon atoms , e.g., formylamino, acetylamino, pivaloylamino, lauroylamino, benzoylamino, 3,4,5-tri-n-octyloxyphenylcarbonylamino" an aminocarbonylamino group [preferably a substituted or unsubstituted aminocarbonylamino group having from 1 to 30 carbon atoms e.g., carbamoylamino, N,N-dimethylaminocarbonylamino, N,N-diethylaminocarbonylamino, morpholinocarbonylamino, an alkoxycarbonylamino group, preferably a substituted or unsubstituted alkoxycarbonylamino group having from 2 to 30 carbon atoms e.g., methoxycarbonylamino, ethoxycarbonylamino, t-butoxycarbonylamino, n-octadecyloxycarbonylamino, N-methylmethoxycarbonylamino, an aryloxycarbonylamino group [preferably a substituted or unsubstituted aryloxycarbonylamino group having from 7 to 30 carbon atoms e.g., phenoxycarbonylamino, p-chlorophenoxycarbonylamino, m-(n-octyloxyphenoxycarbonyl-amino, a sulfamoylamino group , preferably a substituted or unsubstituted sulfamoylamino group having from 0 to 30 carbon atoms e.g., sulfamoylamino, N,N-dimethylaminosulfonylamino, N-n-octylaminosulfonylamino, an alkylsulfonylamino group and arylsulfonylamino group preferably a substituted or unsubstituted alkylsulfonylamino group having from 1 to 30 carbon atoms, a substituted or unsubstituted arylsulfonylamino group having from 6 to 30 carbon atoms (e.g., methylsulfonylamino, butylsulfonylamino, phenylsulfonylamino, 2,3,5-trichlorophenylsulfonylamino, p-methylphenylsulfonyl-amino, a mercapto group, an alkylthio group preferably a substituted or unsubstituted alkylthio group having from 1 to 30 carbon atoms e.g., methylthio, ethylthio, n-hexadecylthio, an arylthio group , preferably a substituted or unsubstituted arylthio group having from 6 to 30 carbon atoms (e.g., phenylthio, p-chlorophenylthio, m-methoxyphenylthio, a heterocyclic thio group , preferably a substituted or unsubstituted heterocyclic thio group having from 2 to 30 carbon atoms e.g., 2-benzothiazolylthio, 1-phenyltetrazol-5-ylthio, a sulfamoyl group [preferably a substituted or unsubstituted sulfamoyl group having from 0 to 30 carbon atoms e.g., N-ethylsulfamoyl, N-(3-dodecyloxypropyl)sulfamoyl, N,N-dimethylsulfamoyl, N-acetylsulfamoyl, N-benzoylsulfamoyl, N-(N'-phenylcarbamoyl)sulfamoyl)], a sulfo group, an alkylsulfinyl group and arylsulfinyl group [preferably a substituted or unsubstituted alkylsulfinyl group having from 1 to 30 carbon atoms, a substituted or unsubstituted arylsulfinyl group having from 6 to 30 carbon atoms e.g., methylsulfinyl, ethylsulfinyl, phenylsulfinyl, p-methylphenylsulfinyl , an alkylsulfonyl group and arylsulfonyl group [preferably a substituted or unsubstituted alkylsulfonyl group having from 1 to 30 carbon atoms, a substituted or unsubstituted arylsulfonyl group having from 6 to 30 carbon atoms e.g., methylsulfonyl, ethylsulfonyl, phenylsulfonyl, p-methylphenylsulfonyl , an acyl group preferably a formyl group, a substituted or unsubstituted alkylcarbonyl group having from 2 to 30 carbon atoms, a substituted or unsubstituted arylcarbonyl group having from 7 to 30 carbon atoms, a substituted or unsubstituted heterocyclic carbonyl group having from 4 to 30 carbon atoms bonded to the carbonyl group via a carbon atom e.g., acetyl, pivaloyl, 2-chloroacetyl, stearoyl, benzoyl, p-n-octyloxyphenylcarbonyl, 2-pyridylcarbonyl, 2-furylcarbonyl, an aryloxycarbonyl group, preferably a substituted or unsubstituted aryloxycarbonyl group having from 7 to 30 carbon atoms e.g., phenoxycarbonyl, o-chlorophenoxycarbonyl, m-nitrophenoxycarbonyl, p-t-butylphenoxycarbonyl , an alkoxycarbonyl group , preferably a substituted or unsubstituted alkoxycarbonyl group having from 2 to 30 carbon atoms e.g., methoxycarbonyl, ethoxycarbonyl, t-butoxycarbonyl, n-octadecyloxycarbonyl , a carbamoyl group preferably a substituted or unsubstituted carbamoyl group having from 1 to 30 carbon atoms e.g., carbamoyl, N-methylcarbamoyl, N,N-dimethylcarbamoyl, N,N-di-n-octylcarbamoyl, N-(methyl-sulfonyl) carbamoyl, an arylazo group and heterocyclic azo group preferably a substituted or unsubstituted arylazo group having from 6 to 30 carbon atoms, a substituted or unsubstituted heterocyclic azo group having from 3 to 30 carbon atoms e.g., phenylazo, p-chlorophenylazo, 5-ethylthio-1,3,4-thiadiazol-2-ylazo, an imido group preferably N-succinimido, N-phthalimido, a phosphino group preferably a substituted or unsubstituted phosphino group having from 2 to 30 carbon atoms e.g., dimethylphosphino, diphenylphosphino, methylphenoxyphosphino , a phosphinyl group preferably a substituted or unsubstituted phosphinyl group having from 2 to 30 carbon atoms (e.g., phosphinyl, dioctyloxyphosphinyl, diethoxyphosphinyl, a phosphinyloxy group preferably a substituted or unsubstituted phosphinyloxy group having from 2 to 30 carbon atoms e.g., diphenoxyphosphinyloxy, dioctyloxyphosphinyloxy, a phosphinylamino group , preferably a substituted or unsubstituted phosphinylamino group having from 2 to 30 carbon atoms e.g., dimethoxyphosphinylamino, dimethylaminophosphinylamino, a phospho group, a silyl group preferably a substituted or unsubstituted silyl group having from 3 to 30 carbon atoms e.g., trimethylsilyl, t-butyldimethylsilyl, phenyldimethylsilyl, a hydrazino group preferably a substituted or unsubstituted hydrazino group having from 0 to 30 carbon atoms e.g., trimethylhydrazino, or a ureido group preferably a substituted or unsubstituted ureido group having from 0 to 30 carbon atoms (e.g., N,N-dimethylureido.

Rings may be condensed. For example, an aromatic or nonaromatic hydrocarbon ring or heterocyclic ring may form a polycyclic condensed ring in combination. Examples of such rings include a benzene ring, a naphthalene ring, an anthracene ring, a quinoline ring, a phenanthrene ring, a fluorene ring, a triphenylene ring, a naphthacene ring, a biphenyl ring, a pyrrole ring, a furan ring, a thiophene ring, an imidazole ring, an oxazole ring, a thiazole ring, a pyridine ring, a pyrazine ring, a pyrimidine ring, a pyridazine ring, an indolizine ring, an indole ring, a benzofuran ring, a benzothiophene ring, an isobenzofuran ring, a quinolizine ring, an isoquinoline ring, a phthalazine ring, a naphthyridine ring, a quinoxaline ring, a quinoxazoline ring, a carbazole ring, a phenanthridine ring, an acridine ring, a phenanthroline ring, a thianthrene ring, a chromene ring, a xanthene ring, a phenoxthine ring, a phenothiazine ring, and a phenazine ring.

The specific examples of the alkyl group, aryl group and heterocyclic group represented by R" include an alkyl group, e.g., an unsubstituted alkyl group having from 1 to 18, preferably from 1 to 6, and particularly preferably from 1 to 4, carbon atoms (e.g., methyl, ethyl, propyl, isopropyl, butyl, isobutyl, hexyl, octyl, dodecyl, octadecyl, and a substituted alkyl group having from 1 to 18, preferably from 1 to 6, and particularly preferably from 1 to 4, carbon atoms e.g., an aralkyl group e.g., benzyl, 2-phenylethyl.

Preferably, the methine direct dye of general formula I is such that X represents CRR' or NR". According to one embodiment, R and R' represent, independently of each other, a hydrogen atom; a linear or branched C₁-C₆ alkyl group, a linear or branched C₁-C₆ alkoxy group; a hydroxyl group; a linear or branched C₁-C₆ hydroxyalkyl group; a nitro group; a sulfo group; a carboxyl group.

Preferably, R" represents a hydrogen atom, a linear or branched C₁-C₆ alkyl group, a hydroxyl group; or a linear or branched C₁-C₆ hydroxyalkyl group, a C₁-C₆alkylsulfonium group.

The present invention encompasses any compositions containing a compound of formula (I) but any of the mesomeric forms of this compounds.

More preferably, the methine direct dye of general formula I is such that X represents NR" and R" represents a hydrogen atom; a linear or branched C₁-C₆ alkyl group, a hydroxyl group; a linear or branched C₁-C₆ hydroxyalkyl group; a C₁-C₆ alkylsulfonium group; or an aryl group.

According to one embodiment, the methine direct dye of general formula I is such that R₁, R₂, R₃ and R₄ represent, independently of each other, a hydrogen atom; a halogen atom; a linear or branched C₁-C₆ alkyl group; a linear or branched C₁-C₆ alkoxy group; a hydroxyl group; a linear or branched C₁-C₆ hydroxyalkyl group ; a nitro group ; a sulfo group ; a carboxy group ; a linear or branched C₁-C₆ alkylcarboxyl group ; a formyl group ; a linear or branched C₁-C₆ alkylcarbonyl group ; a thionyl group ; a group NRaRb in which Ra and Rb, independently of each other, represent a hydrogen atom, a linear or branched C₁-C₆ alkyl group or a linear or branched C₁-C₆ hydroxyalkyl group ; an acetylamino group ; a linear or branched C₁-C₆ alkylcarbonyl group ; a hydrogenocarbonyl group ; a hydroxycarbonyl group ; a linear or branched C₁-C₆ alkoxycarbonyl group ; a substituted or unsubstituted aromatic group ; a substituted or unsubstituted heteroaromatic group ; a substituted or unsubstituted oxyaromatic group ; a substituted or unsubstituted oxyheteroaromatic group, or at least one of the groups R₁, R₂, R₃ and R₄ independently of each other, forms, with the carbon atom to which it is attached, a carbonyl group.

Preferably, at least one of the groups R₁, R₂, R₃ and R₄, independently of each other, forms, with the carbon atom to which it is attached, a carbonyl group.

L₁, L₂ and L₃ each represent a methine group. Each of these methine groups represented by L₁ to L₃ may have a substituent. For example, a substituted or unsubstituted alkyl group having from 1 to 15, preferably from 1 to 10, more preferably from 1 to 5, carbon atoms (e.g., methyl, ethyl, 2-carboxyethyl), a substituted or unsubstituted aryl group having from 6 to 20, preferably from 6 to 15, more preferably from 6 to 10, carbon atoms (e.g., phenyl, o-carboxyphenyl), a substituted or unsubstituted heterocyclic group having from 3 to 20, preferably from 4 to 15, more preferably from 6 to 10, carbon atoms (e.g., N,N-diethylbarbituric acid), a halogen atom (e.g., chlorine, bromine, iodine, fluorine), an alkoxyl group having from 1 to 15, preferably from 1 to 10, more preferably from 1 to 5, carbon atoms (e.g., methoxy, ethoxy), an amino group having from 0 to 15, preferably from 2 to 10, more preferably from 4 to 10, carbon atoms (e.g., methylamino, N,N-dimethylamino, N-methyl-N-phenylamino, N-methylpiperazino), an alkylthio group having from 1 to 15, preferably from 1 to 10, more preferably from 1 to 5, carbon atoms (e.g., methylthio, ethylthio), an arylthio group having from 6 to 20, preferably from 6 to 12, more preferably from 6 to 10, carbon atoms (e.g., phenylthio, p-methylphenylthio) can be exemplified as the substituents. Each of these methine groups may form a ring together with other methine groups

L₁, L₂ and L₃ preferably represent an unsubstituted methine group.

M is included in the formula to show the presence of a cation or an anion when a counter ion is necessary to neutralize the ionic charge of the dye. Representative cations may be either inorganic cations or organic cations and examples include inorganic ions such as a hydrogen ion (H⁺), an alkali metal ion (e.g., a sodium ion, a potassium ion, a lithium ion), and an alkaline earth metal ion (e.g., a calcium ion), and organic ions such as an ammonium ion {preferably an ammonium ion, e.g., an ammonium ion substituted with a substituted or unsubstituted alkyl, aryl or heterocyclic group having from 1 to 30 carbon atoms [preferably a tetraalkylammonium ion (preferably a tetramethylammonium ion), a trialkylammonium ion (preferably a triethylammonium ion), a 1,8-diazabicyclo[5.4.0]-7-undecenium ion], a substituted or unsubstituted cationic heterocyclic ion having from 1 to 30 carbon atoms (preferably a pyridinium ion, an ethylpyridinium ion)}. Anions may be either inorganic anions or organic anions, and examples include inorganic ions such as a halide anion (e.g., a fluorine ion, a chlorine ion, a bromine ion, an iodine ion), a sulfuric acid ion, a perchloric acid ion, and a tetrafluoroboric acid ion, and organic ions such as a substituted arylsulfonic acid ion (e.g., a p-toluenesulfonic acid ion, a p-chlorobenzene-sulfonic acid ion), an aryldisulfonic acid ion (e.g., a 1,3-benzenedisulfonic acid ion, a 1,5-naphthalenedisulfonic acid ion, a 2,6-naphthalenedisulfonic acid ion), an alkylsulfuric acid ion (e.g., a methylsulfuric acid ion), a thiocyanic acid ion, a picric acid ion, an acetic acid ion, and a trifluoro-methanesulfonic acid ion. In addition, ionic polymers or other dyes having a counter charge to the dye may be used. Further, CO₂⁻ and SO₃⁻ can be described as CO₂H and SO₃H when they have hydrogen ions as the counter ions.

M preferably is as the cation, more preferably a hydrogen ion, a sodium ion, a potassium ion and a triethylammmonium ion.

When n represents 2 or more, a methine group is repeated but they need not be the same.

Even more preferably, the methine direct dye corresponds to the formula (II): 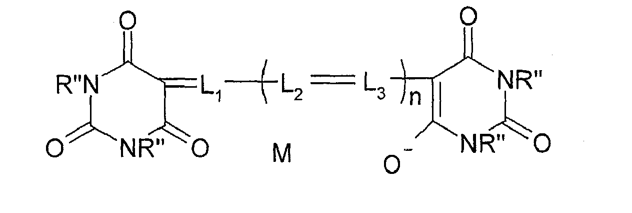 wherein, L1, L2, L3, n, R" and M are as defined for general formula (I).

Preferably R" each independently represents a hydrogen atom; a linear or branched C₁-C₆ alkyl group, a hydroxyl group; a linear or branched C₁-C₆ hydroxyalkyl group; a an C₁-C₆alkylsulfonium group; or an aryl group, n is an integer ranging from 0 to 20 and L1, L2 and L3 represent independently an unsubstituted methine group or a substituted with a linear or branched C₁-C₆ alkyl group; a linear or branched C₁-C₆ alkoxy group; an aryl group.

Preferably, n is from 1 to 10, more preferably from 1 to 4.

According to one embodiment, R" independently of each other, represent a hydrogen atom, a linear or branched C₁-C₆ alkyl group, a hydroxyl group; or a linear or branched C₁-C₆ hydroxyalkyl group,

Examples of methine direct dyes of formula (I) are for example

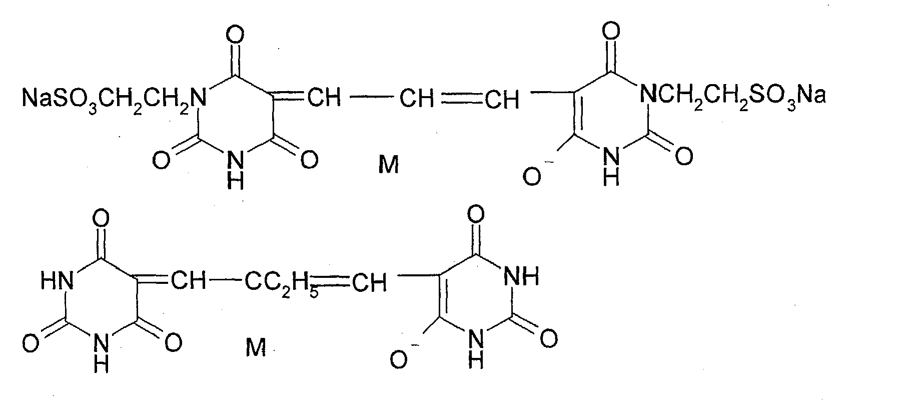

The composition according to the present patent application comprises from 0.0001% to 30%, preferably from 0.001% to 20% and more preferably from 0.01% to 15% by weight of methine direct dye(s) of formula (I) relative to the total weight of the composition.

The dye for use in the present invention can be prepared according to the process described in F. M. Harmer, Heterocyclic Compounds--Cyanine Dyes and Related Compounds, (particularly regarding an oxonol dye, in pp. 463 to 488), John Wiley & Sons, New York, London (1964), D. M. Sturmer, Heterocyclic CompoundsSpecial Topics in Heterocyclic Chemistry, Chap. 18, Clause 14, pp. 482 to 515, John Wiley & Sons, New York, London (1977), Rodd's Chemistry of Carbon Compounds, 2nd Ed., Vol. IV, Part B, Chap. 15, pp. 369 to 422, Elsevier Science Publishing Company Inc., (particularly regarding an oxonol dye, in pp. 410 to 411), New York (1977).

The composition according to the present invention can also contains at least one conditioner, preferably chosen from cationic polymers, cations, silicones, chitosans and chitosan derivatives.

For the purposes of the present invention, the term "cationic polymer" denotes any polymer containing cationic groups and/or groups that may be ionized into cationic groups.

The cationic polymers that may be used in accordance with the present invention may be chosen from all those already known per se as improving the cosmetic properties of the hair, i.e. especially those described in patent application EP-A-337 354 and in French patents FR-2 270 846, 2 383 660, 2 598 611, 2 470 596 and 2 519 863.

The cationic polymers that are preferred are chosen from those containing units comprising primary, secondary, tertiary and/or quaternary amine groups, which may either form part of the main polymer chain or may be borne by a side substituent directly attached thereto.

The cationic polymers used generally have a number-average molecular mass of between 500 and 5 × 10⁶ approximately and preferably between 10³ and 3 × 10⁶ approximately.

Among the cationic polymers that may be mentioned more particularly are polymers of the polyamine, polyamino amide and polyquaternary ammonium type.

These are known products. They are described in particular in French patents Nos 2 505 348 and 2 542 997.

Thus, among these polymers, mention may be made of:
(1) copolymers of acrylamide and of dimethylaminoethyl methacrylate quaternized with dimethyl sulphate or with a dimethyl halide, such as the product sold under the name Hercofloc by the company Hercules,
   - the copolymers of acrylamide and of methacryloyloxyethyltrimethylammonium chloride described, for example, in patent application EP-A-080 976 and sold under the name Bina Quat P 100 by the company Ciba Geigy,
   - the copolymer of acrylamide and of methacryloyloxyethyltrimethylammonium methosulphate sold under the name Reten by the company Hercules,
   - quaternized or non-quaternized vinylpyrrolidone/dialkylaminoalkyl acrylate or methacrylate copolymers, such as the products sold under the name "Gafquat" by the company ISP, such as, for example, "Gafquat 734" or "Gafquat 755", or alternatively the products known as "Copolymer 845, 958 and 937". These polymers are described in detail in French patents 2 077 143 and 2 393 573,
   - dimethylaminoethyl methacrylate/vinylcaprolactam/vinylpyrrolidone terpolymers, such as the product sold under the name Gaffix VC 713 by the company ISP,
   - vinylpyrrolidone/methacrylamidopropyldimethylamine copolymers sold in particular under the name Styleze CC 10 by ISP, and
   - quaternized vinylpyrrolidone/dimethylaminopropyl methacrylamide copolymers such as the product sold under the name "Gafquat HS 100" by the company ISP.
(2) The cellulose ether derivatives containing quaternary ammonium groups, described in French patent 1 492 597, and in particular polymers sold under the names "JR" (JR 400, JR 125 and JR 30M) or "LR" (LR 400 or LR 30M) by the company Union Carbide Corporation. These polymers are also defined in the CTFA dictionary as quaternary ammoniums of hydroxyethylcellulose that have reacted with an epoxide substituted with a trimethylammonium group.
(3) Cationic cellulose derivatives such as cellulose copolymers or cellulose derivatives grafted with a water-soluble quaternary ammonium monomer, and described in particular in US patent 4 131 576, such as hydroxyalkylcelluloses, for instance hydroxymethyl-, hydroxyethyl- or hydroxypropylcelluloses grafted, in particular, with a methacryloylethyltrimethylammonium, methacrylamidopropyltrimethylammonium or dimethyldiallylammonium salt.
   The commercial products corresponding to this definition are more particularly the products sold under the names "Celquat L 200" and "Celquat H 100" by the company National Starch.
(4) The cationic polysaccharides described more particularly in US patents 3 589 578 and 4 031 307, such as guar gums containing cationic trialkylammonium groups. Guar gums modified with a salt (e.g. chloride) of 2,3-epoxypropyltrimethylammonium are used, for example.
   Such products are sold in particular under the trade names Jaguar C13 S, Jaguar C 15, Jaguar C 17 and Jaguar C162 by the company Meyhall.
(5) Polymers consisting of piperazinyl units and of divalent alkylene or hydroxyalkylene radicals containing straight or branched chains, optionally interrupted by oxygen, sulphur or nitrogen atoms or by aromatic or heterocyclic rings, as well as the oxidation and/or quaternization products of these polymers. Such polymers are described, in particular, in French patents 2 162 025 and 2 280 361.
(6) Water-soluble polyamino amides prepared in particular by polycondensation of an acidic compound with a polyamine; these polyamino amides can be crosslinked with an epihalohydrin, a diepoxide, a dianhydride, an unsaturated dianhydride, a bis-unsaturated derivative, a bis-halohydrin, a bis-azetidinium, a bis-haloacyldiamine, a bis-alkyl halide or alternatively with an oligomer resulting from the reaction of a difunctional compound which is reactive with a bis-halohydrin, a bis-azetidinium, a bis-haloacyldiamine, a bis-alkyl halide, an epihalohydrin, a diepoxide or a bis-unsaturated derivative; the crosslinking agent being used in proportions ranging from 0.025 to 0.35 mol per amine group of the polyamino amide; these polyamino amides can be alkylated or, if they contain one or more tertiary amine functions, they can be quaternized. Such polymers are described, in particular, in French patents 2 252 840 and 2 368 508.
(7) The polyamino amide derivatives resulting from the condensation of polyalkylene polyamines with polycarboxylic acids followed by alkylation with difunctional agents. Mention may be made, for example, of adipic acid/dialkylaminohydroxyalkyldialkylenetriamine polymers in which the alkyl radical contains from 1 to 4 carbon atoms and preferably denotes methyl, ethyl or propyl. Such polymers are described in particular in French patent 1 583 363.
   Among these derivatives, mention may be made more particularly of the adipic acid/dimethylaminohydroxypropyl/diethylenetriamine polymers sold under the name Cartaretine F, F4 or F8 by the company Sandoz.
(8) The polymers obtained by reaction of a polyalkylene polyamine containing two primary amine groups and at least one secondary amine group with a dicarboxylic acid chosen from diglycolic acid and saturated aliphatic dicarboxylic acids having from 3 to 8 carbon atoms. The molar ratio between the polyalkylene polyamine and the dicarboxylic acid is between 0.8:1 and 1.4:1; the polyamino amide resulting therefrom is reacted with epichlorohydrin in a molar ratio of epichlorohydrin relative to the secondary amine group of the polyamino amide of between 0.5:1 and 1.8:1. Such polymers are described in particular in US patents 3 227 615 and 2 961 347.
   Polymers of this type are sold in particular under the name Hercosett 57 by the company Hercules Inc. or alternatively under the name PD 170 or Delsette 101 by the company Hercules in the case of the adipic acid/epoxypropyl/diethylenetriamine copolymer.
(9) Cyclopolymers of alkyldiallylamine or of dialkyldiallylammonium.
   These polymers are described in particular in French patent 2 080 759 and in its Certificate of Addition 2 190 406.
   Among the polymers defined above, mention may be made more particularly of the dimethyldiallylammonium chloride homopolymer sold under the name Merquat 100 by the company Calgon (and its homologs of low weight-average molecular mass) and the copolymers of diallyldimethylammonium chloride and of acrylamide sold under the name Merquat 550.
(10) Diquaternary ammonium polymers, as described especially in French patents 2 320 330, 2 270 846, 2 316 271, 2 336 434 and 2 413 907 and US patents 2 273 780, 2 375 853, 2 388 614, 2 454 547, 3 206 462, 2 261 002, 2 271 378, 3 874 870, 4 001 432, 3 929 990, 3 966 904, 4 005 193, 4 025 617, 4 025 627, 4 025 653, 4 026 945 and 4 027 020.
   It is more particularly possible to use polymers that consist of repeating units corresponding to formula (XIII) below: 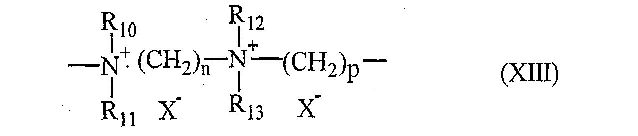 in which R₁₀, R₁₁, R₁₂ and R₁₃, which may be identical or different, denote an alkyl or hydroxyalkyl radical containing from 1 to 4 carbon atoms approximately, n and p are integers ranging from 2 to 20 approximately, and X⁻ is an anion derived from a mineral or organic acid.
(11) Polyquaternary ammonium polymers consisting, for example, of those prepared according to the processes described in US patents 4 157 388, 4 702 906 and 4 719 282. They are especially described in patent application EP-A-122 324.
   Among these products, mention may be made, for example, of Mirapol A 15, Mirapol AD1, Mirapol AZ1 and Mirapol 175 sold by the company Miranol.
(12) Quaternary polymers of vinylpyrrolidone and of vinylimidazole, such as, for example, the products sold under the names Luviquat FC 905, FC 550 and FC 370 by the company BASF.
(13) Polyamines such as Polyquart H sold by Henkel, which is given under the reference name "Polyethylene glycol (15) tallow polyamine" in the CTFA dictionary.
(14) Crosslinked methacryloyloxy(C₁-C₄)alkyltri(C₁-C₄)-alkylammonium salt polymers such as the polymers obtained by homopolymerization of dimethylaminoethyl methacrylate quaternized with methyl chloride, or by copolymerization of acrylamide with dimethylaminoethyl methacrylate quaternized with methyl chloride, the homo- or copolymerization being followed by crosslinking with a compound containing olefinic unsaturation, in particular methylenebisacrylamide. A crosslinked acrylamide/methacryloyloxyethyltrimethylammonium chloride copolymer (20/80 by weight) in the form of a dispersion containing 50% by weight of the said copolymer in mineral oil can be used more particularly. This dispersion is sold under the name Salcare® SC 92 by the company Allied Colloids. A crosslinked methacryloyloxyethyltrimethylammonium chloride homopolymer containing about 50% by weight of the homopolymer in mineral oil or in a liquid ester can also be used. These dispersions are sold under the names Salcare® SC 95 and Salcare® SC 96 by the company Allied Colloids.

Other cationic polymers that can be used in the context of the invention are polyalkyleneimines, in particular polyethyleneimines, polymers containing vinylpyridine or vinylpyridinium units, condensates of polyamines and of epichlorohydrin, quaternary polyureylenes and chitin derivatives.

Among all the cationic polymers that may be used in the context of the present invention, it is preferred to use the polymers of families (1), (9), (10), (11) and (14) and even more preferably the polymers containing repeating units of formulae (W) and (U) below: 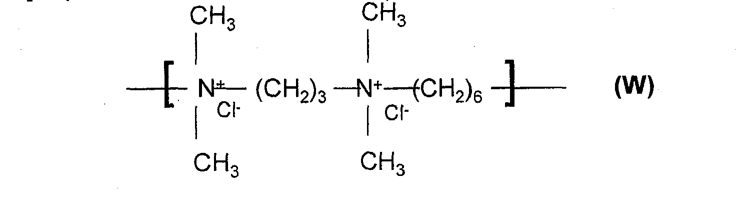 and in particular those whose molecular weight, determined by gel permeation chromatography, is between 9500 and 9900; 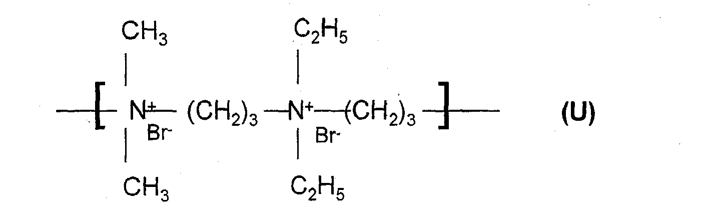 and especially those whose molecular weight, determined by gel permeation chromatography, is about 1200.

The concentration of cationic polymer in the composition according to the present invention may range from 0.01% to 10%, preferably from 0.05% to 5% and even more preferably from 0.1% to 3% by weight relative to the total weight of the composition.

The silicones that may be used in the compositions according to the present invention may be linear, cyclic, branched or unbranched, and volatile or non-volatile. They may be in the form of oils, resins or gums, and may in particular be polyorganosiloxanes that are insoluble in the cosmetically acceptable medium.

The organopolysiloxanes are defined in greater detail in Walter Noll's "Chemistry and Technology of Silicones" (1968) Academic Press. They can be volatile or non-volatile.

When they are volatile, the silicones are more particularly chosen from those having a boiling point of between 60°C and 260°C, and even more particularly from:
(i) cyclic silicones containing from 3 to 7 and preferably 4 to 5 silicon atoms. These are, for example, octamethylcyclotetrasiloxane sold in particular under the name Volatile Silicone 7207 by Union Carbide or Silbione 70045 V 2 by Rhodia Chimie, decamethylcyclopentasiloxane sold under the name Volatile Silicone 7158 by Union Carbide, and Silbione 70045 V 5 by Rhodia, and mixtures thereof.
   Mention may also be made of cyclocopolymers of the dimethylsiloxane/methylalkylsiloxane type, such as Volatile Silicone FZ 3109 sold by the company Union Carbide, having the chemical structure: 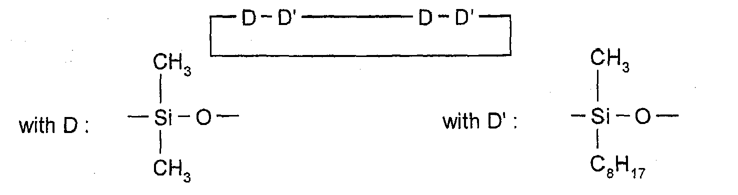 Mention may also be made of mixtures of cyclic silicones with organosilicon compounds, such as the mixture of octamethylcyclotetrasiloxane and tetratrimethylsilylpentaerythritol (50/50) and the mixture of octamethylcyclotetrasiloxane and oxy-1,1'-bis(2,2,2',2',3,3'-hexatrimethylsilyloxy)neopentane;
(ii) linear volatile silicones containing 2 to 9 silicon atoms and having a viscosity of less than or equal to 5×10⁻⁶ m²/s at 25°C. An example is decamethyltetrasiloxane sold in particular under the name SH 200 by the company Toray Silicone. Silicones belonging to this category are also described in the article published in Cosmetics and Toiletries, Vol. 91, Jan. 76, pp. 27-32, Todd & Byers "Volatile Silicone Fluids for Cosmetics".

Among the non-volatile silicones that may be mentioned especially are polyalkylsiloxanes, polyarylsiloxanes, polyalkylarylsiloxanes, silicone gums and resins, and polyorganosiloxanes modified with organofunctional groups, and mixtures thereof.

The organomodified silicones that may be used in accordance with the invention are silicones as defined above and containing in their structure one or more organofunctional groups attached via a hydrocarbon-based group.

Among the organomodified silicones that may be mentioned are polyorganosiloxanes containing:
- polyethyleneoxy and/or polypropyleneoxy groups optionally containing C₆-C₂₄ alkyl groups, such as the products known as dimethicone copolyol sold by the company Dow Corning under the name DC 1248 or the oils Silwet® L 722, L 7500, L 77 and L 711 from the company Union Carbide and the (C₁₂)alkylmethicone copolyol sold by the company Dow Corning under the name Q2 5200;
- substituted or unsubstituted amine groups, such as the products sold under the name GP 4 Silicone Fluid and GP 7100 by the company Genesee, or the products sold under the names Q2 8220 and Dow Corning 929 or 939 by the company Dow Corning. The substituted amine groups are, in particular, C₁-C₄ aminoalkyl groups;
- thiol groups such as the products sold under the names "GP 72 A" and "GP 71" from Genesee;
- alkoxylated groups such as the product sold under the name "Silicone Copolymer F-755" by SWS Silicones and Abil Wax® 2428, 2434 and 2440 by the company Goldschmidt;
- hydroxylated groups such as the polyorganosiloxanes containing a hydroxyalkyl function, described in French patent application FR-A-85/16334;
- acyloxyalkyl groups such as, for example, the polyorganosiloxanes described in patent US-A-4 957 732;
- anionic groups of the carboxylic acid type, such as, for example, in the products described in patent EP 186 507 from the company Chisso Corporation, or of alkylcarboxylic type, such as those present in the product X-22-3701E from the company Shin-Etsu; 2-hydroxyalkyl sulphonate; 2-hydroxyalkyl thiosulphate such as the products sold by the company Goldschmidt under the names "Abil® S201" and "Abil® S255";
- hydroxyacylamino groups, such as the polyorganosiloxanes described in patent application EP 342 834. Mention may be made, for example, of the product Q2-8413 from the company Dow Corning.

The silicone oils that may be used in the compositions according to the invention are volatile or non-volatile polymethylsiloxanes containing a linear or cyclic silicone chain, which are liquid or pasty at room temperature, especially cyclopolydimethylsiloxanes (cyclomethicones) such as cyclohexasiloxane; polydimethylsiloxanes comprising alkyl, alkoxy or phenyl groups, which are pendent or at the end of a silicone chain, these groups containing from 2 to 24 carbon atoms; phenylsilicones, for instance phenyltrimethicones, phenyldimethicones, phenyltrimethylsiloxydiphenylsiloxanes, diphenyldimethicones, diphenylmethyldiphenyltrisiloxanes, 2-phenylethyltrimethyl siloxysilicates, polymethylphenylsiloxanes; and mixtures thereof.

The silicone gums that may be used in the compositions according to the invention are polydiorganosiloxanes with a high molecular mass, of between 200 000 and 2 000 000, used alone or as a mixture in a solvent chosen from volatile silicones, polydimethylsiloxane oils, polyphenylmethylsiloxane oils, polydiphenyldimethylsiloxane oils, isoparaffins, methylene chloride, pentane and hydrocarbons, or mixtures thereof.

A silicone gum with a molecular weight of less than 1 500 000 is preferably used. The silicone gums are, for example, polydimethylsiloxanes, polyphenylmethylsiloxanes, poly(diphenylsiloxanedimethylsiloxanes), poly(dimethylsiloxanemethylvinylsiloxanes), poly(dimethylsiloxanephenylmethylsiloxanes) or poly(diphenylsiloxanedimethylsiloxanemethylvinylsiloxanes).

These silicone gums may be terminated at a chain end with trimethylsilyl or dimethylhydroxysilyl groups.

The silicone resins that may be used in the compositions according to the invention are crosslinked siloxane systems containing units R₂SiO_{2/2}, RSiO_{3/2} or SiO_{4/2}, in which R represents a hydrocarbon-based group containing from 1 to 6 carbon atoms or a phenyl group. Among these products, the ones that are particularly preferred are those in which R denotes a lower (C₁-C₆) alkyl radical or a phenyl radical.

The chitosans and chitosan derivatives that may be used are in particular chitosan salts such as chitosan acetate, lactate, glutamate, gluconate or pyrrolidonecarboxylate.

Among these compounds, mention may be made of chitosan having a degree of deacetylation of 90.5% by weight, sold under the name Kytan Brut Standard by the company Aber Technologies, and chitosan pyrrolidonecarboxylate sold under the name Kytamer® PC by the company Amerchol.

The concentration of conditioner(s) in the composition according to the present invention may range from 0.01% to 10%, preferably from 0.05% to 5% and even more preferably from 0.1% to 3% by weight relative to the total weight of the composition.

According to a second preferred mode, the composition according to the present invention also contains at least one thickening polymer, also known as a "rheology modifier".

The rheology modifiers may be chosen from fatty acid amides (coconut diethanolamide or monoethanolamide, or oxyethylenated monoethanolamide of carboxylic acid alkyl ether), cellulose-based thickeners (hydroxyethylcellulose, hydroxypropylcellulose or carboxymethylcellulose), guar gum and its derivatives (hydroxypropylguar), gums of microbial origin (xanthan gum, scleroglucan gum), crosslinked homopolymers of acrylic acid or of acrylamidopropanesulphonic acid, and associative polymers as described below.

The associative polymers that may be used according to the invention are water-soluble polymers capable, in an aqueous medium, of reversibly combining with each other or with other molecules.

Their chemical structure comprises hydrophilic zones and hydrophobic zones characterized by at least one fatty chain.

The associative polymers that may be used according to the invention may be of anionic, cationic, amphoteric or, preferably, nonionic type.

Their weight concentration in the dye composition may range from about 0.01% to 10% of the total weight of the composition, and in the ready-to-use composition (comprising the oxidizing agent), from about 0.0025% to 10% of the total weight of the composition. More preferably, this amount ranges from about 0.1% to 5% by weight in the dye composition and from about 0.025% to 10% in the ready-to-use composition.

Among the associative polymers of anionic type that may be mentioned are:
- (1) those comprising at least one hydrophilic unit and at least one fatty-chain allyl ether unit, more particularly those whose hydrophilic unit consists of an ethylenic unsaturated anionic monomer, more particularly of a vinylcarboxylic acid and most particularly of an acrylic acid or a methacrylic acid or mixtures thereof. Anionic associative polymers of this type are described and prepared, according to an emulsion polymerization process, in patent EP-0 216 479.
   Among these anionic associative polymers that are particularly preferred are crosslinked terpolymers of methacrylic acid, of ethyl acrylate and of polyethylene glycol (10 EO) stearyl alcohol ether (Steareth-10), in particular those sold by the company Allied Colloids under the names Salcare SC 80® and Salcare SC 90® , which are aqueous 30% emulsions of a crosslinked terpolymer of methacrylic acid, of ethyl acrylate and of steareth-10 allyl ether (40/50/10).
- (II) those comprising at least one hydrophilic unit of unsaturated olefinic carboxylic acid type, and at least one hydrophobic unit of (C₁₀-C₃₀)alkyl ester of unsaturated carboxylic acid type.
   Preferably, these polymers are chosen from those in which the hydrophilic unit of unsaturated olefinic carboxylic acid type corresponds to acrylic acid, methacrylic acid or ethacrylic acid units, and in which the hydrophobic unit is of (C₁₀-C₃₀)alkyl ester of unsaturated carboxylic acid type containing C₁₀-C₃₀ and preferably C₁₂-C₂₂ alkyl acrylate, methacrylate or ethacrylate units.
   (C₁₀-C₃₀) alkyl esters of unsaturated carboxylic acids according to the invention include, for example, lauryl acrylate, stearyl acrylate, decyl acrylate, isodecyl acrylate and dodecyl acrylate, and the corresponding methacrylates, lauryl methacrylate, stearyl methacrylate, decyl methacrylate, isodecyl methacrylate and dodecyl methacrylate.
   Anionic polymers of this type are described and prepared, for example, according to US patents 3 915 921 and 4 509 949.
   Among the anionic associative polymers of this type that will be used more particularly are polymers formed from a monomer mixture comprising:
   (i) essentially acrylic acid,
   (ii) an alkyl acrylate ester containing from 12 to 22 carbon atoms, and
   (iii) a crosslinking agent, which is a well-known copolymerizable unsaturated polyethylenic monomer, for instance diallyl phthalate, allyl (meth)acrylate, divinylbenzene, (poly)ethylene glycol dimethacrylate and methylenebisacrylamide.
   Among anionic associative polymers of this type that will be used more particularly are those consisting of from 95% to 60% by weight of acrylic acid (hydrophilic unit), 4% to 40% by weight of C₁₀-C₃₀ alkyl acrylate (hydrophobic unit) and 0% to 6% by weight of crosslinking polymerizable monomer, or alternatively those consisting of from 98% to 96% by weight of acrylic acid (hydrophilic unit), 1% to 4% by weight of C₁₀-C₃₀ alkyl acrylate (hydrophobic unit) and 0.1% to 0.6% by weight of crosslinking polymerizable monomer such as those described above.
   Among the said above polymers, those most particularly preferred according to the present invention are the products sold by the company Goodrich under the trade names Pemulen TR1® , Pemulen TR2® and Carbopol 1382® , and even more preferentially Pemulen TR1® , and the product sold by the company SEPPIC under the name Coatex SX® .
- (III) maleic anhydride/C₃₀-C₃₈ α-olefin/alkyl maleate terpolymers, such as the product (maleic anhydride/C₃₀-C₃₈ α-olefin/isopropyl maleate copolymer) sold under the name Performa V 1608® by the company Newphase Technologies.
- (IV) acrylic terpolymers comprising:
   (a) about 20% to 70% by weight of a carboxylic acid containing α,β-monoethylenic unsaturation,
   (b) about 20% to 80% by weight of a non-surfactant monomer containing α,β-monoethylenic unsaturation other than (a),
   (c) about 0.5% to 60% by weight of a nonionic monourethane which is the product of reaction of a monohydric surfactant with a monoisocyanate containing monoethylenic unsaturation,
   such as those described in patent application EP-A-0 173 109 and more particularly the terpolymer described in Example 3, namely a methacrylic acid/methyl acrylate/behenyl dimethyl-meta-isopropenylbenzylisocyanate ethoxylated (40 EO) terpolymer, as an aqueous 25% dispersion.
- (V) copolymers comprising among their monomers a carboxylic acid containing α,β-monoethylenic unsaturation and an ester of a carboxylic acid containing α,β-monoethylenic unsaturation and of an oxyalkylenated fatty alcohol.

Preferentially, these compounds also comprise as monomer an ester of a carboxylic acid containing α,β-monoethylenic unsaturation and of a C₁-C₄ alcohol.

An example of a compound of this type that may be mentioned is Aculyn 22® sold by the company Rohm & Haas, which is a methacrylic acid/ethyl acrylate/stearyl methacrylate oxyalkylenated terpolymer.

Among the associative polymers of cationic type that may be mentioned are:
- (I) cationic associative polyurethanes, the family of which has been described by the Applicant in French patent application FR 2 811 993; it may be represented by the general formula (XVIII) below:

   R-X-(P)ₙ-[L-(Y)ₘ]ᵣ-L'-(P')ₚ-X'-R' (XVIII)

   in which:
   R and R', which may be identical or different, represent a hydrophobic group or a hydrogen atom;
   X and X', which may be identical or different, represent a group comprising an amine function optionally bearing a hydrophobic group, or alternatively a group L";
   L, L' and L", which may be identical or different, represent a group derived from a diisocyanate;
   P and P', which may be identical or different, represent a group comprising an amine function optionally bearing a hydrophobic group;
   Y represents a hydrophilic group;
   r is an integer between 1 and 100, preferably between 1 and 50 and in particular between 1 and 25;
   n, m and p each range, independently of each other, from 0 to 1000;
   the molecule containing at least one protonated or quaternized amine function and at least one hydrophobic group.
   In one preferred embodiment of these polyurethanes, the only hydrophobic groups are the groups R and R' at the chain ends.
   One preferred family of cationic associative polyurethanes is the one corresponding to formula (XVIII) described above and in which:
   R and R' both independently represent a hydrophobic group,
   X and X' each represent a group L",
   n and p are between 1 and 1000, and
   L, L', L", P, P', Y and m have the meaning given above.
   Another preferred family of cationic associative polyurethanes is the one corresponding to formula (XVIII) above in which:
   R and R' both independently represent a hydrophobic group, X and X' each represent a group L", n and p are 0, and L, L', L", Y and m have the meaning given above.
   The fact that n and p are 0 means that these polymers do not comprise units derived from a monomer containing an amine function, incorporated into the polymer during the polycondensation. The protonated amine functions of these polyurethanes result from the hydrolysis of excess isocyanate functions, at the chain end, followed by alkylation of the primary amine functions formed with alkylating agents containing a hydrophobic group, i.e. compounds of the type RQ or R'Q, in which R and R' are as defined above and Q denotes a leaving group such as a halide, a sulphate, etc.
   Yet another preferred family of cationic associative polyurethanes is the one corresponding to formula (XVIII) above in which:
   R and R' both independently represent a hydrophobic group,
   X and X' both independently represent a group comprising a quaternary amine,
   n and p are zero, and
   L, L', Y and m have the meaning given above.
   The number-average molecular mass of the cationic associative polyurethanes is preferably between 400 and 500 000, in particular between 1000 and 400 000 and ideally between 1000 and 300 000.
   The expression "hydrophobic group" means a radical or polymer containing a saturated or unsaturated, linear or branched hydrocarbon-based chain, which may contain one or more hetero atoms such as P, O, N or S, or a radical containing a perfluoro or silicone chain. When the hydrophobic group denotes a hydrocarbon-based radical, it comprises at least 10 carbon atoms, preferably from 10 to 30 carbon atoms, in particular from 12 to 30 carbon atoms and more preferably from 18 to 30 carbon atoms.
   Preferentially, the hydrocarbon-based group is derived from a monofunctional compound.
   By way of example, the hydrophobic group may be derived from a fatty alcohol such as stearyl alcohol, dodecyl alcohol or decyl alcohol. It may also denote a hydrocarbon-based polymer such as, for example, polybutadiene.
   As regards the meaning of Y, the term "hydrophilic group" means a polymeric or non-polymeric water-soluble group.
   By way of example, when it is not a polymer, mention may be made of ethylene glycol, diethylene glycol and propylene glycol.
   When it is a hydrophilic polymer, in accordance with one preferred embodiment, mention may be made, for example, of polyethers, sulphonated polyesters, sulphonated polyamides or a mixture of these polymers. The hydrophilic compound is preferentially a polyether and in particular a poly(ethylene oxide) or poly(propylene oxide).
   Although the presence of a hydrophilic group Y is optional, cationic associative polyurethanes comprising such a group are, however, preferred.
- (II) quaternized cellulose derivatives and polyacrylates containing non-cyclic amine side groups.
   The quaternized cellulose derivatives are, in particular:
   - quaternized celluloses modified with groups comprising at least one fatty chain, such as alkyl, arylalkyl or alkylaryl groups containing at least 8 carbon atoms, or mixtures thereof.
   - quaternized hydroxyethylcelluloses modified with groups comprising at least one fatty chain, such as alkyl, arylalkyl or alkylaryl groups containing at least 8 carbon atoms, or mixtures thereof.
   The alkyl radicals borne by the above quaternized celluloses or hydroxyethylcelluloses preferably contain from 8 to 30 carbon atoms. The aryl radicals preferably denote phenyl, benzyl, naphthyl or anthryl groups.
   Examples of quaternized alkylhydroxyethylcelluloses containing C₈-C₃₀ fatty chains that may be mentioned include the products Quatrisoft LM 200® , Quatrisoft LM-X 529-18-A® , Quatrisoft LM-X 529-18B® (C₁₂ alkyl) and Quatrisoft LM-X 529-8® (C₁₈ alkyl) sold by the company Amerchol, and the products Crodacel QM® , Crodacel QL® (C₁₂ alkyl) and Crodacel QS® (C₁₈ alkyl) sold by the company Croda.

### Amphoteric associative polymers

The amphoteric associative polymers are preferably chosen from those comprising at least one non-cyclic cationic unit. Even more particularly, the ones that are preferred are those prepared from or comprising 1 to 20 mol%, preferably 1.5 to 15 mol% and even more particularly 1.5 to 6 mol% of fatty-chain monomer relative to the total number of moles of monomers.

The amphoteric associative polymers that are preferred according to the invention comprise or are prepared by copolymerizing:
1) at least one monomer (XIX) or (XX): [lacuna]
2) at least one monomer (XXI) [lacuna]
   and
3) at least one monomer (XXII): [lacuna]

The monomers (XIX) and (XX) are, for example:
- dimethylaminoethyl methacrylate, dimethylaminoethyl acrylate,
- diethylaminoethyl methacrylate, diethylaminoethyl acrylate,
- dimethylaminopropyl methacrylate, dimethylaminopropyl acrylate,
- dimethylaminopropylmethacrylamide, dimethylaminopropylacrylamide,
these monomers optionally being quaternized, for example with a C₁-C₄ alkyl halide or a C₁-C₄ dialkyl sulphate.

More particularly, the monomer of formula (XIX) is chosen from acrylamidopropyltrimethylammonium chloride and methacrylamidopropyltrimethylammonium chloride.

The monomers of formula (XXI) of the present invention are for example acrylic acid, methacrylic acid, crotonic acid and 2-methylcrotonic acid. More particularly, the monomer of formula (XXI) is acrylic acid.

The monomers of formula (XXII) are preferably chosen from the group consisting of C₁₂-C₂₂ and more particularly C₁₆-C₁₈ alkyl acrylates or methacrylates.

The monomers constituting the fatty-chain amphoteric polymers of the invention are preferably already neutralized and/or quaternized.

The ratio of the number of cationic charges/anionic charges is preferably equal to about 1.

The amphoteric associative polymers according to the invention preferably comprise from 1 mol% to 10 mol% of the monomer comprising a fatty chain (monomer of formula (XIX), (XX) or (XXII)), and preferably from 1.5 mol% to 6 mol%.

The weight-average molecular weights of the amphoteric associative polymers according to the invention may range from 500 to 50 000 000 and are preferably between 10 000 and 5 000 000.

The amphoteric associative polymers according to the invention may also contain other monomers such as nonionic monomers and in particular such as C₁-C₄ alkyl acrylates or methacrylates.

Amphoteric associative polymers according to the invention are described and prepared, for example, in patent application WO 98/44012.

Among the amphoteric associative polymers according to the invention, the ones that are preferred are acrylic acid/(meth)acrylamidopropyltrimethylammonium chloride/stearyl methacrylate terpolymers.

The associative polymers of nonionic type used according to the invention are preferably chosen from:
- (1) celluloses modified with groups comprising at least one fatty chain;
   examples that may be mentioned include:
   - hydroxyethylcelluloses modified with groups comprising at least one fatty chain, such as alkyl, arylalkyl or alkylaryl groups, or mixtures thereof, and in which the alkyl groups are preferably C₈-C₂₂, for instance the product Natrosol Plus Grade 330 CS® (C₁₆ alkyls) sold by the company Aqualon, or the product Bermocoll EHM 100® sold by the company Berol Nobel,
   - those modified with alkylphenyl polyalkylene glycol ether groups, such as the product Amercell Polymer HM-1500® (nonylphenyl polyethylene glycol (15) ether) sold by the company Amerchol.
- (2) hydroxypropyl guars modified with groups comprising at least one fatty chain, such as the product Esaflor HM 22® (C₂₂ alkyl chain) sold by the company Lamberti, and the products RE210-18® (C₁₄ alkyl chain) and RE205-1® (C₂₀ alkyl chain) sold by the company RhônePoulenc.
- (3) copolymers of vinylpyrrolidone and of fatty-chain hydrophobic monomers; examples that may be mentioned include:

- the products Antaron V216® or Ganex V216® (vinylpyrrolidone/hexadecene copolymer) sold by the company I.S.P.
- the products Antaron V220® or Ganex V220® (vinylpyrrolidone/eicosene copolymer) sold by the company I.S.P.
- (4) copolymers of C₁-C₆ alkyl methacrylates or acrylates and of amphiphilic monomers comprising at least one fatty chain, such as, for example, the oxyethylenated methyl acrylate/stearyl acrylate copolymer sold by the company Goldschmidt under the name Antil 208® .
- (5) copolymers of hydrophilic methacrylates or acrylates and of hydrophobic monomers comprising at least one fatty chain, such as, for example, the polyethylene glycol methacrylate/lauryl methacrylate copolymer.
- (6) polyurethane polyethers comprising in their chain both hydrophilic blocks usually of polyoxyethylenated nature and hydrophobic blocks which may be aliphatic sequences alone and/or cycloaliphatic and/or aromatic sequences.
- (7) polymers with an aminoplast ether skeleton containing at least one fatty chain, such as the Pure Thix® compounds sold by the company Sud-Chemie.

Preferably, the polyurethane polyethers comprise at least two hydrocarbon-based lipophilic chains containing from 6 to 30 carbon atoms, separated by a hydrophilic block, the hydrocarbon-based chains possibly being pendent chains, or chains at the end of the hydrophilic block. In particular, it is possible for one or more pendent chains to be included. In addition, the polymer may comprise a hydrocarbon-based chain at one end or at both ends of a hydrophilic block.

The polyurethane polyethers may be multiblock, in particular in triblock form. Hydrophobic blocks may be at each end of the chain (for example: triblock copolymer with a hydrophilic central block) or distributed both at the ends and in the chain (for example: multiblock copolymer). These same polymers may also be graft polymers or starburst polymers.

The nonionic fatty-chain polyurethane polyethers may be triblock copolymers in which the hydrophilic block is a polyoxyethylenated chain comprising from 50 to 1 000 oxyethylene groups. The nonionic polyurethane polyethers comprise a urethane linkage between the hydrophilic blocks, whence arises the name.

By extension, also included among the nonionic fatty-chain polyurethane polyethers are those in which the hydrophilic blocks are linked to the lipophilic blocks via other chemical bonds.

As examples of nonionic fatty-chain polyurethane polyethers that may be used in the invention, it is also possible to use Rheolate 205® containing a urea function, sold by the company Rheox, or Rheolate® 208, 204 or 212, and also Acrysol RM 184® .

Mention may also be made of the product Elfacos T210® containing a C₁₂₋₁₄ alkyl chain, and the product Elfacos T212® containing a C₁₈ alkyl chain, from Akzo.

The product DW 1206B® from Rohm & Haas containing a C₂₀ alkyl chain and a urethane linkage, sold at a solids content of 20% in water, may also be used.

It is also possible to use solutions or dispersions of these polymers, especially in water or in aqueous-alcoholic medium. Examples of such polymers that may be mentioned are Rheolate® 255, Rheolate® 278 and Rheolate® 244 sold by the company Rheox. The products DW 1206F and DW 1206J sold by the company Rohm & Haas may also be used.

The polyurethane polyethers that may be used according to the invention are in particular those described in the article by G. Fonnum, J. Bakke and Fk. Hansen - Colloid Polym. Sci 271, 380.389 (1993).

It is even more particularly preferred to use a polyurethane polyether that may be obtained by polycondensation of at least three compounds comprising (i) at least one polyethylene glycol comprising from 150 to 180 mol of ethylene oxide, (ii) stearyl alcohol or decyl alcohol, and (iii) at least one diisocyanate.

Such polyurethane polyethers are sold especially by the company Rohm & Haas under the names Aculyn 46® and Aculyn 44® [Aculyn 46® is a polycondensate of polyethylene glycol containing 150 or 180 mol of ethylene oxide, of stearyl alcohol and of methylenebis(4-cyclohexyl isocyanate) (SMDI), at 15% by weight in a matrix of maltodextrin (4%) and water (81%); Aculyn 44® is a polycondensate of polyethylene glycol containing 150 or 180 mol of ethylene oxide, of decyl alcohol and of methylenebis(4-cyclohexylisocyanate) (SMDI), at 35% by weight in a mixture of propylene glycol (39%) and water (26%)].

According to a third preferred mode, the composition according to the present invention also contains at least one surfactant.

The surfactants that are suitable for carrying out the present invention are especially the following:

### (i) Anionic surfactant(s):

By way of example of anionic surfactants that can be used, alone or as mixtures, in the context of the present invention, mention may be made in particular (non-limiting list) of salts (in particular alkali metal salts, especially sodium salts, ammonium salts, amine salts, amino alcohol salts or magnesium salts) of the following compounds: alkyl sulphates, alkyl ether sulphates, alkylamido ether sulphates, alkylarylpolyether sulphates, monoglyceride sulphates; alkyl sulphonates, alkyl phosphates, alkylamide sulphonates, alkylaryl sulphonates, α-olefin sulphonates, paraffin sulphonates; (C₆-C₂₄)alkyl sulphosuccinates, (C₆-C₂₄)alkyl ether sulphosuccinates, (C₆₋C₂₄)alkylamide sulphosuccinates; (C₆-C₂₄)alkyl sulphoacetates; (C₆₋C₂₄)acyl sarcosinates; and (C₆-C₂₄)acyl glutamates. It is also possible to use (C₆-C₂₄)alkylpolyglycoside carboxylic esters such as alkylglucoside citrates, alkylpolyglycoside tartrates and alkylpolyglycoside sulphosuccinates, alkylsulphosuccinamates; acyl isethionates and N-acyl taurates, the alkyl or acyl radical of all of these different compounds preferably containing from 12 to 20 carbon atoms and the aryl radical preferably denoting a phenyl or benzyl group. Among the anionic surfactants which can also be used, mention may also be made of fatty acid salts such as oleic, ricinoleic, palmitic and stearic acid salts, coconut oil acid or hydrogenated coconut oil acid; acyl lactylates in which the acyl radical contains 8 to 20 carbon atoms. It is also possible to use alkyl D-galactoside uronic acids and their salts, polyoxyalkylenated (C₆-C₂₄)alkyl ether carboxylic acids, polyoxyalkylenated (C₆-C₂₄)alkylaryl ether carboxylic acids, polyoxyalkylenated (C₆-C₂₄)alkylamido ether carboxylic acids and their salts, in particular those containing from 2 to 50 alkylene oxide groups, in particular ethylene oxide groups, and mixtures thereof.

### (ii) Nonionic surfactant(s):

The nonionic surfactants are, themselves also, compounds that are well known per se (see in particular in this respect "Handbook of Surfactants" by M.R. Porter, published by Blackie & Son (Glasgow and London), 1991, pp. 116-178) and their nature is not a critical factor in the context of the present invention. Thus, they can be chosen in particular from (non-limiting list) polyethoxylated or polypropoxylated, alkylphenols, alpha-diols or alcohols, having a fatty chain containing, for example, 8 to 18 carbon atoms, it being possible for the number of ethylene oxide or propylene oxide groups to range in particular from 2 to 50. Mention may also be made of copolymers of ethylene oxide and of propylene oxide, condensates of ethylene oxide and of propylene oxide with fatty alcohols; polyethoxylated fatty amides preferably having from 2 to 30 mol of ethylene oxide, polyglycerolated fatty amides containing on average 1 to 5, and in particular 1.5 to 4, glycerol groups; oxyethylenated fatty acid esters of sorbitan having from 2 to 30 mol of ethylene oxide; fatty acid esters of sucrose, fatty acid esters of polyethylene glycol, alkylpolyglycosides, N-alkylglucamine derivatives, and amine oxides such as (C₁₀-C₁₄)alkylamine oxides or N-acylaminopropylmorpholine oxides.

### (iii) Amphoteric or zwitterionic surfactant(s):

The amphoteric or zwitterionic surfactants, the nature of which is not a critical factor in the context of the present invention, can be, in particular (non-limiting list), aliphatic secondary or tertiary amine derivatives in which the aliphatic radical is a linear or branched chain containing 8 to 18 carbon atoms and containing at least one water-solubilizing anionic group (for example carboxylate, sulphonate, sulphate, phosphate or phosphonate); mention may also be made of (C₈-C₂₀)alkylbetaines, sulphobetaines, (C₈-C₂₀)alkylamido(C₁-C₆)-alkylbetaines or (C₈-C₂₀)alkylamido(C₁-C₆)alkylsulphobetaines.

Among the amine derivatives, mention may be made of the products sold under the name Miranol, as described in US patents 2 528 378 and 2 781 354 and classified in the CTFA dictionary, 3rd edition, 1982, under the names Amphocarboxyglycinates and Amphocarboxypropionates.

These compounds are classified in the CTFA dictionary, 5th edition, 1993, under the names Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphopropionate, Disodium Caprylamphodipropionate, Disodium Caprylamphodipropionate, Lauroamphodipropionic acid and Cocoamphodipropionic acid.

By way of example, mention may be made of the cocoamphodiacetate sold under the trade name Miranol® C2M concentrate by the company Rhodia Chimie.

### (iv) Cationic surfactants:

Among the cationic surfactants, mention may be made in particular (non-limiting list) of: salts of optionally polyoxyalkylenated primary, secondary or tertiary fatty amines; quaternary ammonium salts such as tetraalkylammonium, alkylamidoalkyltrialkylammonium, trialkylbenzylammonium, trialkylhydroxyalkylammonium or alkylpyridinium chlorides or bromides; imidazoline derivatives; or amine oxides of cationic nature.

The amounts of surfactants present in the composition according to the invention can range from 0.01% to 40% and preferably from 0.5% to 30% relative to the total weight of the composition.

The composition of the present invention may also comprise one or more oxidation dye precursors: one or more oxidation bases and/or one or more couplers. By way of example, the oxidation bases are chosen from para-phenylenediamines, bis(phenyl)alkylenediamines, para-aminophenols, ortho-aminophenols and heterocyclic bases and the addition salts thereof.

Among the para-phenylenediamines that may be mentioned, for example, are para-phenylenediamine, para-tolylenediamine, 2-chloro-para-phenylenediamine, 2,3-dimethyl-para-phenylenediamine, 2,6-dimethyl-para-phenylenediamine, 2,6-diethyl-para-phenylenediamine, 2,5-dimethyl-para-phenylenediamine, N,N-dimethyl-para-phenylenediamine, N,N-diethyl-para-phenylenediamine, N,N-dipropyl-para-phenylenediamine, 4-amino-N,N-diethyl-3-methylaniline, N,N-bis(β-hydroxyethyl)-para-phenylenediamine, 4-N,N-bis(β-hydroxyethyl)-amino-2-methylaniline, 4-N,N-bis(β-hydroxyethyl)amino-2-chloroaniline, 2-β-hydroxyethyl-para-phenylenediamine, 2-fluoro-para-phenylenediamine, 2-isopropyl-para-phenylenediamine, N-(β-hydroxypropyl)-para-phenylenediamine, 2-hydroxymethyl-para-phenylenediamine, N,N-dimethyl-3-methyl-para-phenylenediamine, N-ethyl-N-(β-hydroxyethyl)-para-phenylenediamine, N-(β,γ-dihydroxypropyl)-para-phenylenediamine, N-(4'-aminophenyl)-para-phenylenediamine, N-phenyl-para-phenylenediamine, 2-β-hydroxyethyloxy-para-phenylenediamine, 2-β-acetylaminoethyloxy-para-phenylenediamine, N-(β-methoxyethyl)-para-phenylenediamine, 4-aminophenylpyrrolidine, 2-thienyl-para-phenylenediamine, 2-β-hydroxyethylamino-5-aminotoluene and 3-hydroxy-1-(4'-aminophenyl)pyrrolidine, and the addition salts thereof with an acid.

Among the para-phenylenediamines mentioned above, para-phenylenediamine, para-tolylenediamine, 2-isopropyl-para-phenylenediamine, 2-β-hydroxyethyl-para-phenylenediamine, 2-β-hydroxyethyloxy-para-phenylenediamine, 2,6-dimethyl-para-phenylenediamine, 2,6-diethyl-para-phenylenediamine, 2,3-dimethyl-para-phenylenediamine, N,N-bis(β-hydroxyethyl)-para-phenylenediamine, 2-chloro-para-phenylenediamine and 2-β-acetylaminoethyloxy-para-phenylenediamine, and the addition salts thereof with an acid, are particularly preferred.

Among the bis(phenyl)alkylenediamines that may be mentioned, for example, are N,N'-bis(β-hydroxyethyl)-N,N'-bis(4'-aminophenyl)-1,3-diaminopropanol, N,N'-bis(β-hydroxyethyl)-N,N'-bis(4'-aminophenyl)ethylenediamine, N,N'-bis(4-aminophenyl)tetramethylenediamine, N,N'-bis(β-hydroxyethyl)-N,N'-bis(4-aminophenyl)tetramethylenediamine, N,N'-bis(4-methylaminophenyl)tetramethylenediamine, N,N'-bis(ethyl)-N,N'-bis(4'-amino-3'-methylphenyl)-ethylenediamine and 1,8-bis(2,5-diaminophenoxy)-3,6-dioxaoctane, and the addition salts thereof.

Among the para-aminophenols that may be mentioned, for example, are para-aminophenol, 4-amino-3-methylphenol, 4-amino-3-fluorophenol, 4-amino-3-chlorophenol, 4-amino-3-hydroxymethylphenol, 4-amino-2-methylphenol, 4-amino-2-hydroxymethylphenol, 4-amino-2-methoxymethylphenol, 4-amino-2-aminomethylphenol, 4-amino-2-(β-hydroxyethylaminomethyl)phenol and 4-amino-2-fluorophenol, and the addition salts thereof with an acid.

Among the ortho-aminophenols that may be mentioned, for example, are 2-aminophenol, 2-amino-5-methylphenol, 2-amino-6-methylphenol and 5-acetamido-2-aminophenol, and the addition salts thereof.

Among the heterocyclic bases that may be mentioned, for example, are pyridine derivatives, pyrimidine derivatives and pyrazole derivatives.

Among the pyridine derivatives that may be mentioned are the compounds described, for example, in patents GB 1 026 978 and GB 1 153 196, for instance 2,5-diaminopyridine, 2-(4-methoxyphenyl)-amino-3-aminopyridine, and 3,4-diaminopyridine, and the addition salts thereof.

Other pyridine oxidation bases that are useful in the present invention are the 3-aminopyrazolo[1,5-a]pyridine oxidation bases or addition salts thereof described, for example, in patent application FR 2 801 308. Examples that may be mentioned include pyrazolo[1,5-a]pyrid-3-ylamine, 2-acetylaminopyrazolo[1,5-a]pyrid-3-ylamine, 2-morpholin-4-ylpyrazolo[1,5-a]pyrid-3-ylamine, 3-aminopyrazolo-[1,5-a]pyridine-2-carboxylic acid, 2-methoxypyrazolo[1,5-a]pyrid-3-ylamino, (3-aminopyrazolo[1,5-a]pyrid-7-yl)methanol, 2-(3-aminopyrazolo[1,5-a]pyrid-5-yl)ethanol, 2-(3-aminopyrazolo[1,5-a]pyrid-7-yl)ethanol, (3-aminopyrazolo[1,5-a]pyrid-2-yl)methanol, 3,6-diaminopyrazolo[1,5-a]pyridine, 3,4-diaminopyrazolo[1,5-a]pyridine, pyrazolo[1,5-a]pyridine-3,7-diamine, 7-morpholin-4-ylpyrazolo[1,5-a]pyrid-3-ylamine, pyrazolo[1,5-a]pyridine-3,5-diamine, 5-morpholin-4-ylpyrazolo[1,5-a]pyrid-3-ylamine, 2-[(3-aminopyrazolo[1,5-a]pyrid-5-yl)(2-hydroxyethyl)amino]ethanol, 2-[(3-aminopyrazolo[1,5-a]pyrid-7-yl)(2-hydroxyethyl)amino]ethanol, 3-aminopyrazolo[1,5-a]pyridin-5-ol, 3-aminopyrazolo[1,5-a]pyridin-4-ol, 3-aminopyrazolo[1,5-a]pyridin-6-ol and 3-aminopyrazolo[1,5-a]pyridin-7-ol,
and the addition salts thereof.

Among the pyrimidine derivatives that may be mentioned are the compounds described, for example, in patents DE 2 359 399; JP 88-169 571; JP 05-63124; EP 0 770 375 or patent application WO 96/15765, for instance 2,4,5,6-tetraaminopyrimidine, 4-hydroxy-2,5,6-triaminopyrimidine, 2-hydroxy-4,5,6-triaminopyrimidine, 2,4-dihydroxy-5,6-diaminopyrimidine and 2,5,6-triaminopyrimidine, and the addition salts thereof, and the tautomeric forms thereof, when a tautomeric equilibrium exists.

Among the pyrazole derivatives that may be mentioned are the compounds described in patents DE 3 843 892 and DE 4 133 957, and patent applications WO 94/08969, WO 94/08970, FR-A-2 733 749 and DE 195 43 988, for instance 4,5-diamino-1-methylpyrazole, 4,5-diamino-1-(β-hydroxyethyl)pyrazole, 3,4-diaminopyrazole, 4,5-diamino-1-(4'-chlorobenzyl)pyrazole, 4,5-diamino-1,3-dimethylpyrazole, 4,5-diamino-3-methyl-1-phenylpyrazole, 4,5-diamino-1-methyl-3-phenylpyrazole, 4-amino-1,3-dimethyl-5-hydrazinopyrazole, 1-benzyl-4,5-diamino-3-methylpyrazole, 4,5-diamino-3-tert-butyl-1-methylpyrazole, 4,5-diamino-1-tert-butyl-3-methylpyrazole, 4,5-diamino-1-(β-hydroxyethyl)-3-methylpyrazole, 4,5-diamino-1-ethyl-3-methylpyrazole, 4,5-diamino-1-ethyl-3-(4'-methoxyphenyl)pyrazole, 4,5-diamino-1-ethyl-3-hydroxymethylpyrazole, 4,5-diamino-3-hydroxymethyl-1-methylpyrazole, 4,5-diamino-3-hydroxymethyl-1-isopropylpyrazole, 4,5-diamino-3-methyl-1-isopropylpyrazole, 4-amino-5-(2'-aminoethyl)amino-1,3-dimethylpyrazole, 3,4,5-triaminopyrazole, 1-methyl-3,4,5-triaminopyrazole, 3,5-diamino-1-methyl-4-methylaminopyrazole and 3,5-diamino-4-(β-hydroxyethyl)amino-1-methylpyrazole, and the addition salts thereof.

The additional oxidation base(s) present in the composition of the invention is (are) generally present in an amount ranging from 0.001% to 20% by weight approximately, and preferably ranging from 0.005% to 6%, relative to the total weight of the dye composition.

The composition according to the invention preferably contains one or more couplers conventionally used for dyeing keratin fibres. Among these couplers, mention may be made especially of meta-phenylenediamines, meta-diphenols, naphthalene-based couplers and heterocyclic couplers, and the addition salts thereof.

Examples that may be mentioned include 1,3-dihydroxybenzene, 1,3-dihydroxy-2-methylbenzene, 4-chloro-1,3-dihydroxybenzene, 2,4-diamino-1-(β-hydroxyethyloxy)benzene, 2-amino-4-(β-hydroxyethylamino)-1-methoxybenzene, 1,3-diaminobenzene, 1,3-bis(2,4-diaminophenoxy)propane, 3-ureidoaniline, 3-ureido-1-dimethylaminobenzene, sesamol, 1-β-hydroxyethylamino-3,4-methylenedioxybenzene, α-naphthol, 2-methyl-1-naphthol, 6-hydroxyindole, 4-hydroxyindole, 4-hydroxy-N-methylindole, 2-amino-3-hydroxypyridine, 6-hydroxybenzomorpholine, 3,5-diamino-2,6-dimethoxypyridine, 1-N-(β-hydroxyethyl)amino-3,4-methylenedioxybenzene and 2,6-bis(β-hydroxyethylamino)toluene, and the addition salts thereof.

In the composition of the present invention, the coupler(s) is (are) generally present in an amount ranging from 0.001% to 20% and preferably ranging from 0.005% to 6% by weight approximately relative to the total weight of the dye composition.

In general, the addition salts of the oxidation bases and couplers that may be used in the context of the invention are chosen especially from the addition salts with an acid, such as the hydrochlorides, hydrobromides, sulphates, citrates, succinates, tartrates, lactates, tosylates, benzenesulphonates, phosphates and acetates, and the addition salts with a base, such as sodium hydroxide, potassium hydroxide, ammonia, amines or alkanolamines.

The dye composition in accordance with the invention may also contain one or more additional direct dyes differing from the direct (methine) dyes of formula I, which may especially be chosen from neutral, acidic or cationic nitrobenzene direct dyes, neutral, acidic or cationic azo direct dyes, neutral, acidic or cationic quinone and in particular anthraquinone direct dyes, azine direct dyes, triarylmethane direct dyes, indoamine direct dyes and natural direct dyes.

Among the benzenic direct dyes that may be used according to the invention, mention may be made, in a non-limiting manner, of the following compounds:
1,4-diamino-2-nitrobenzene
1-amino-2-nitro-4-(β-hydroxyethylamino)benzene
1-amino-2-nitro-4-bis(β-hydroxyethyl)aminobenzene
1,4-bis(β-hydroxyethylamino)-2-nitrobenzene
1-β-hydroxyethylamino-2-nitro-4-bis(β-hydroxyethylamino)-benzene
1-β-hydroxyethylamino-2-nitro-4-aminobenzene
1-β-hydroxyethylamino-2-nitro-4-(ethyl)(β-hydroxyethyl)-aminobenzene
1-amino-3-methyl-4-β-hydroxyethylamino-6-nitrobenzene
1-amino-2-nitro-4-β-hydroxyethylamino-5-chlorobenzene
1,2-diamino-4-nitrobenzene
1-amino-2-β-hydroxyethylamino-5-nitrobenzene
1,2-bis(β-hydroxyethylamino)-4-nitrobenzene
1-amino-2-[tris(hydroxymethyl)methylamino]-5-nitrobenzene
1-hydroxy-2-amino-5-nitrobenzene
1-hydroxy-2-amino-4-nitrobenzene
1-hydroxy-3-nitro-4-aminobenzene
1-hydroxy-2-amino-4,6-dinitrobenzene
1-β-hydroxyethyloxy-2-β-hydroxyethylamino-5-nitrobenzene
1-methoxy-2-β-hydroxyethylamino-5-nitrobenzene
1-β-hydroxyethyloxy-3-methylamino-4-nitrobenzene
1-β,γ-dihydroxypropyloxy-3-methylamino-4-nitrobenzene
1-β-hydroxyethylamino-4-β,γ-dihydroxypropyloxy-2-nitrobenzene
1-β,γ-dihydroxypropylamino-4-trifluoromethyl-2-nitrobenzene
1-β-hydroxyethylamino-4-trifluoromethyl-2-nitrobenzene
1-β-hydroxyethylamino-3-methyl-2-nitrobenzene
1-β-aminoethylamino-5-methoxy-2-nitrobenzene
1-hydroxy-2-chloro-6-ethylamino-4-nitrobenzene
1-hydroxy-2-chloro-6-amino-4-nitrobenzene
1-hydroxy-6-[bis(β-hydroxyethyl)amino]-3-nitrobenzene
1-β-hydroxyethylamino-2-nitrobenzene
1-hydroxy-4-β-hydroxyethylamino-3-nitrobenzene.

Among the azo direct dyes that may be used according to the invention, mention may be made of the cationic azo dyes described in patent applications WO 95/15144, WO 95/01772 and EP 714 954, the content of which forms an integral part of the invention.

Among these compounds, mention may be made most particularly of the following dyes:
1,3-dimethyl-2-[[4-(dimethylamino)phenyl]azo]-1H-imidazolium chloride,
1,3-dimethyl-2-[(4-aminophenyl)azo]-1H-imidazolium chloride,
1-methyl-4-[(methylphenylhydrazono)methyl]pyridinium methyl sulphate.

Among the azo direct dyes that may also be mentioned are the following dyes described in the Colour Index International 3rd edition:
Disperse Red 17
Acid Yellow 9
Acid Black 1
Basic Red 22
Basic Red 76
Basic Yellow 57
Basic Brown 16
Acid Yellow 36
Acid Orange 7
Acid Red 33
Acid Red 35
Basic Brown 17
Acid Yellow 23
Acid Orange 24
Disperse Black 9.

Mention may also be made of 1-(4'-aminodiphenylazo)-2-methyl-4-[bis(β-hydroxyethyl)amino]benzene and 4-hydroxy-3-(2-methoxyphenylazo)-1-naphthalenesulphonic acid.

Among the quinone direct dyes that may be mentioned are the following dyes:
Disperse Red 15
Solvent Violet 13
Acid Violet 43
Disperse Violet 1
Disperse Violet 4
Disperse Blue 1
Disperse Violet 8
Disperse Blue 3
Disperse Red 11
Acid Blue 62
Disperse Blue 7
Basic Blue 22
Disperse Violet 15
Basic Blue 99
and also the following compounds:
   1-N-methylmorpholiniumpropylamino-4-hydroxyanthraquinone
   1-aminopropylamino-4-methylaminoanthraquinone
   1-aminopropylaminoanthraquinone
   5-β-hydroxyethyl-1,4-diaminoanthraquinone
   2-aminoethylaminoanthraquinone
   1,4-bis(β,γ-dihydroxypropylamino)anthraquinone.

Among the azine dyes that may be mentioned are the following compounds:
Basic Blue 17
Basic Red 2.

Among the triarylmethane dyes that may be used according to the invention, mention may be made of the following compounds:
Basic Green 1
Acid Blue 9
Basic Violet 3
Basic Violet 14
Basic Blue 7
Acid Violet 49
Basic Blue 26
Acid Blue 7.

Among the indoamine dyes that may be used according to the invention, mention may be made of the following compounds:
2-β-hydroxyethylamino-5-[bis(β-4'-hydroxyethyl)amino]-anilino-1,4-benzoquinone;
2-β-hydroxyethylamino-5-(2'-methoxy-4'-amino)anilino-1,4-benzoquinone;
3-N(2' -chloro-4'-hydroxy)phenylacetylamino-6-methoxy-1,4-benzoquinoneimine;
3-N(3'-chloro-4'-methylamino)phenylureido-6-methyl-1,4-benzoquinoneimine;
3-[4'-N-(ethylcarbamylmethyl)amino]phenylureido-6-methyl-1,4-benzoquinoneimine.

Among the natural direct dyes that may be used according to the invention, mention may be made of lawsone, juglone, alizarin, purpurin, carminic acid, kermesic acid, purpurogallin, protocatechaldehyde, indigo, isatin, curcumin, spinulosin and apigenidin. Extracts or decoctions containing these natural dyes may also be used, and especially henna-based poultices or extracts.

The direct dye(s) preferably represent(s) from 0.001% to 20% by weight approximately, and even more preferably from 0.005% to 10% by weight approximately, relative to the total weight of the ready-to-use composition.

The composition according to the invention may also contain at least one hydroxylated solvent, for instance ethanol, propylene glycol, glycerol, polyol monoethers or benzyl alcohol.

It may also contain a non-hydroxylated solvent.

The hydroxylated solvents and non-hydroxylated solvents are preferably present in proportions preferably of between 1% and 40% by weight approximately and even more preferably between 5% and 30% by weight approximately relative to the total weight of the dye composition.

The dye composition in accordance with the invention may also contain various adjuvants conventionally used in hair dye compositions, such as antioxidants, penetrating agents, sequestering agents, fragrances, buffers, dispersants, pH regulators, film-forming agents, ceramides, preserving agents and opacifiers.

The above adjuvants are generally present in an amount for each of between 0.01% and 20% by weight relative to the weight of the composition.

Needless to say, a person skilled in the art will take care to select this or these optional additional compound(s) such that the advantageous properties intrinsically associated with the oxidation dye composition in accordance with the invention are not, or are not substantially, adversely affected by the envisaged addition(s).

The pH of the dye composition in accordance with the invention is generally between 2 and 12 approximately and preferably between 3 and 11 approximately. It may be adjusted to the desired value by means of acidifying or basifying agents for modifying pH usually used for dyeing keratin fibres, or alternatively using standard buffer systems.

Among the acidifying agents that may be mentioned, for example, are mineral or organic acids other than carboxylic diacids such as hydrochloric acid, orthophosphoric acid, sulphuric acid, carboxylic acids, for instance acetic acid, tartaric acid, citric acid or lactic acid, and sulphonic acids.

Among the basifying agents that may be mentioned, for example, are aqueous ammonia, alkaline carbonates, alkanolamines such as monoethanolamine, diethanolamine and triethanolamine and derivatives thereof, sodium hydroxide, potassium hydroxide and the compounds of formula (XXIII) below: 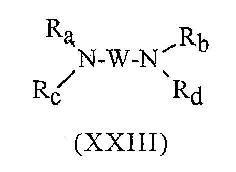 in which W is a propylene residue optionally substituted with a hydroxyl group or a C₁-C₄ alkyl radical; Rₐ, R_{b}, R_{c} and R_{d}, which may be identical or different, represent a hydrogen atom or a C₁-C₄ alkyl or C₁-C₄ hydroxyalkyl radical.

The dye composition according to the invention may be in various forms, such as in the form of liquids, creams or gels, or in any other form that is suitable for dyeing keratin fibres, and especially human hair.

The use of the process according to the present patent application includes the steps of applying the dye composition according to the present patent application to the keratin fibres and then leaving it to act for a period that is sufficient to dye the hair, this period generally being between 5 minutes and 1 hour and preferably between 15 minutes and 1 hour.

When the composition according to the invention comprises at least one oxidation dye precursor, the process for dyeing keratin fibres includes a step in which the colour is developed using an oxidizing agent. The colour may be developed at acidic, neutral or alkaline pH and the oxidizing agent may be added to the composition of the invention just at the time of use, or it may be introduced using an oxidizing composition containing it, applied simultaneously with or sequentially to the composition of the invention.

The oxidizing agents conventionally used for the oxidation dyeing of keratin fibres are, for example, hydrogen peroxide, urea peroxide, alkali metal bromates, persalts such as perborates and persulphates, peracids, and oxidase enzymes, among which mention may be made of peroxidases, 2-electron oxidoreductases such as uricases, and 4-electron oxygenases, for instance laccases. Hydrogen peroxide is particularly preferred.

The oxidizing composition may also contain various adjuvants conventionally used in hair dye compositions and as defined above.

The composition of the invention may be in various forms, such as in the form of liquids, creams or gels, or in any other form that is suitable for dyeing keratin fibres, and especially human hair.

The example that follows illustrates the invention, without, however, being limiting in nature.

### EXAMPLES

### EXAMPLE 1

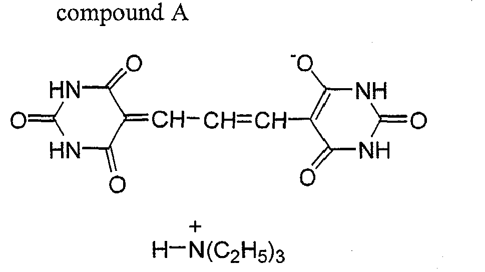

20g(0.156 mole) of barbituric acid, 20g(0.078 mole) of glutaconedialdehidedianile hydrochloride and 65ml(0.47mole) of triethylamine were added to 1000ml of N,N-dimethylformamide and stirred for 5 minutes at room temperature. Then, 18ml (0.19mol) of acetic anhydride were added to the reaction mixture and stirred for 3 hours at room temperature. 2000ml of methanol were added to the reaction mixture and stirred for 1 hour at room temperature. The obtained crystal was filtered. This crystal was added to 1000ml of methanol, refluxed for 1 hour, and filtered after cooling at room temperature. 23.3g of compound A was obtained (yield;76%).
Absorption wavelength (intensity) in MeOH; λmax=485nm(ε=1.25×10⁴)

### EXAMPLE 2

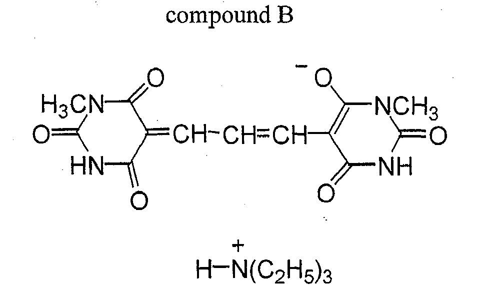

10g(0.070 mole) of N-methylbarbituric acid, 9g(0.035 mole) of glutaconedialdehidedianile hydrochloride and 29ml(0.21mole) of triethylamine were added to 500ml of N,N-dimethylformamide and stirred for 5 minutes at room temperature. Then, 8ml (0.084mol) of acetic anhydride were added to the reaction mixture and stirred for 3 hours at room temperature. 1000ml of methanol were added to the reaction mixture and stirred for 1 hour at room temperature. The obtained crystal was filtered. This crystal was added to 600ml of methanol, refluxed for 1 hour, and filtered after cooling at room temperature. 10.3g of compound A was obtained (yield;70%).
Absorption wavelength (intensity) in MeOH; λmax=486nm(ε=1.21 × 10⁴)

### Example of a composition according to the invention :

The following dyeing composition was prepared

| | |
|---|---|
| Methine direct dye of formula B | 10⁻³ mol% |
| Hydroxyethylcellulose sold by the company | 0.384% |
| Aqualon under the name Natrosol250MR | |
| Mixture of methyl, ethyl, propyl, butyl and isobutyl p-hydroxybenzoates sold by the company NIPA under the name NIPA ester 82121 | 0.032% |
| Alkyl(C8/C10 50/50)polyglucoside sold by the company SEPPIC under the name Oramix CG110 | 5% |
| Benzyl alcohol | 4% |
| Propylene glycol | 6% |
| Demineralized water qs | 100 |

This dye composition was applied on permed hair already dyed with a Majirel black type coloration for 1 hour at 60°C.

After rinsing, shampooing and drying under a hood for 30 minutes, the hair has a coloration that is very visible on dark hair, and very attractive. This show that the coloration obtained with the composition of the invention is highly visible even on dark hair without the need of a pre-bleaching step of the hair.

## Claims

1. A dye composition for dyeing keratin fibres, comprising, in a suitable dyeing medium, at least one methine direct dye of general formula (I): 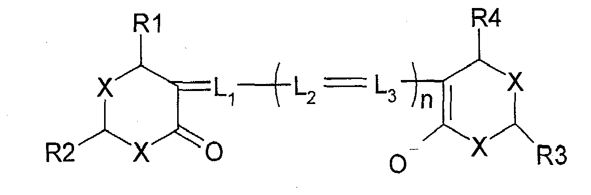 in which X represents CRR', NR", S or O, and
R et R' represent, independently of each other, a hydrogen atom; a halogen atom such as chlorine, bromine, fluorine or iodine; a linear or branched alkyl group substituted or unsubstituted, a linear or branched alkoxy group; a hydroxyl group; a linear or branched hydroxyalkyl group; a nitro group; a sulfo group; a carboxyl group; an acetylamino group; a cyano group; a thiol group; a linear or branched alkylthiol group; a linear or branched thioalkyl group; a linear or branched alkylcarbonyl group; a hydrogenocarbonyl group; a hydroxycarbonyl group; a linear or branched alkoxycarbonyl group; an ammonio group, a mono-, di- or trialkylammonio group; an imidazolio group; a pyridinio group; a substituted or unsubstituted aromatic group; a substituted or unsubstituted heteroaromatic group; a substituted or unsubstituted oxyaromatic group; a substituted or unsubstituted oxyheteroaromatic group; or, R represents a hydrogen atom and R' represents NRaRb in which Ra and Rb, independently of each other, represent a hydrogen atom; a linear or branched C₁-C₆ alkyl group or a linear or branched C₁-C₆ hydroalkyl group;
R" represents a hydrogen atom, a linear or branched alkyl group substituted or not, a hydroxyl group; or a linear or branched hydroxyalkyl group; an alkylsulfonium group such as -(CH₂)₂₆SO₃Na group or an aryl group or a heteroaryl group;
R₁, R₂, R₃ and R₄ represent, independently of each other, a hydrogen atom; a halogen atom; a linear or branched alkyl group substituted or unsubstituted; a linear or branched alkoxy group; a hydroxyl group; a linear or branched hydroxyalkyl group; a nitro group; a sulfo group; a carboxyl group; a linear or branched alkylcarboxyl group, a formyl group; a linear or branched alkylcarbonyl group; a thionyl group; a group NRaRb in which Ra and Rb, independently of each other, represent a hydrogen atom; a linear or branched C₁-C₆ alkyl group or a linear or branched C₁-C₆ hydroxyalkyl group; an acetylamino group; a cyano group; a thiol group; a C₁-C₆ alkylthiol group; a linear or branched thioalkyl group, a linear or branched C₁-C₆ alkylcarbonyl group; a hydrogenocarbonyl group; a hydroxycarbonyl group; a linear or branched alkoxycarbonyl group; an ammonio group; a mono-, di- or tri(C₁-C₆)alkylammonio group; an imidazolio group; a pyridinio group; a substituted or unsubstituted aromatic group; a substituted or unsubstituted heteroaromatic group; a substituted or unsubstituted oxyaromatic group; a substituted or unsubstituted oxyheteroaromatic group; or at least one of the groups R₁, R₂, R₃ and R₄ independently of each other, forms, with the carbon atom to which it is attached, a carbonyl group;
n is an integer ranging from 0 to 20;
L1, L2 and L3 independently represent a substituted or unsubstituted methine group, and
M is the counterion necessary to balance the charge of compound of formula (I).

2. The dye composition according to Claim 1 wherein the methine direct dye of general formula I is such that X represents CRR' or NR".

3. The dye composition according to Claim 1 or 2, wherein R and R' represent, independently of each other, a hydrogen atom; a linear or branched C₁-C₆ alkyl group, a linear or branched C₁-C₆ alkoxy group; a hydroxyl group; a linear or branched C₁-C₆ hydroxyalkyl group; a nitro group; a sulfo group; a carboxyl group.

4. The dye composition according to any of claims 1 to 4 wherein R" represents a hydrogen atom, a linear or branched C₁-C₆ alkyl group, a hydroxyl group; or a linear or branched C₁-C₆ hydroxyalkyl group, a C₁-C₆alkylsulfonium group.

5. The dye composition according to any of Claims 1 to 4, wherein the methine direct dye of general formula I is such that R₁, R₂, R₃ and R₄ represent, independently of each other, a hydrogen atom; a halogen atom; a linear or branched C₁-C₆ alkyl group; a linear or branched C₁-C₆ alkoxy group; a hydroxyl group; a linear or branched C₁-C₆ hydroxyalkyl group; a nitro group; a sulfo group; a carboxyl group; a linear or branched C₁-C₆ alkylcarbonyl group; a formyl group; a linear or branched C₁-C₆ alkylcarboxyl group; a thionyl group; a group NRaRb in which Ra and Rb, independently of each other, represent a hydrogen atom; a linear or branched C₁-C₆ alkyl group or a linear or branched C₁-C₆ hydroxyalkyl group; an acetylamino group; a linear or branched C₁-C₆ alkylcarbonyl group; a hydrogenocarbonyl group; a hydroxycarbonyl group; a linear or branched C₁-C₆ alkoxycarbonyl group; a substituted or unsubstituted aromatic group; a substituted or unsubstituted heteroaromatic group; a substituted or unsubstituted oxyaromatic group; a substituted or unsubstituted oxyheteroaromatic group;
or at least one of the groups R₁, R₂, R₃ and R₄ independently of each other, forms, with the carbon atom to which it is attached, a carbonyl group.

6. The dye composition according to one of Claims 1 to 5, wherein at least one of the groups R₁, R₂, R₃ and R₄, R₅ and R₆, independently of each other, forms, with the carbon atom to which it is attached, a carbonyl group.

7. The dye composition according to any of claims 1 to 6 wherein L₁, L₂ and L₃ preferably represent an unsubstituted methine group.

8. The dye composition according to any of claims 1 to 7, wherein the methine direct dye corresponds to the formula : 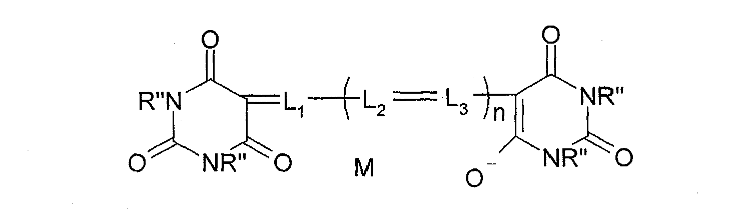 wherein, L1, L2, L3, n, R" and M are as defined in the preceding claims.

9. The dye composition according to claim 8 wherein R" each independently represents a hydrogen atom; a linear or branched C₁-C₆ alkyl group, a hydroxyl group; a linear or branched C₁-C₆ hydroxyalkyl group; a an C₁-C₆alkylsulfonium group; or an aryl group, n is an integer ranging from 0 to 20 and L1, L2 and L3 represent independently an unsubstituted methine group or a substituted with a linear or branched C₁-C₆ alkyl group; a linear or branched C₁-C₆ alkoxy group; an aryl group.

10. The dye composition according to Claim 8 or 9 wherein the methine direct dye is chosen from the compounds corresponding to the formulae: 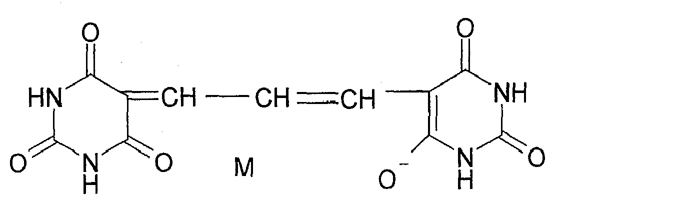 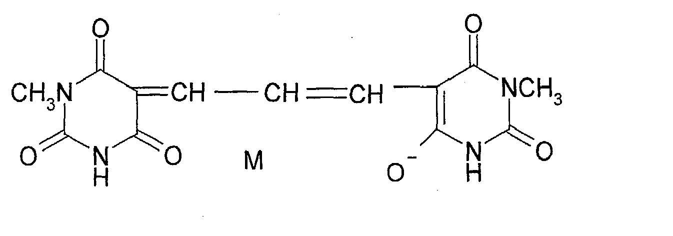 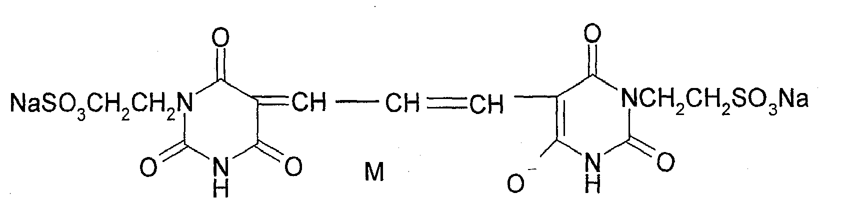 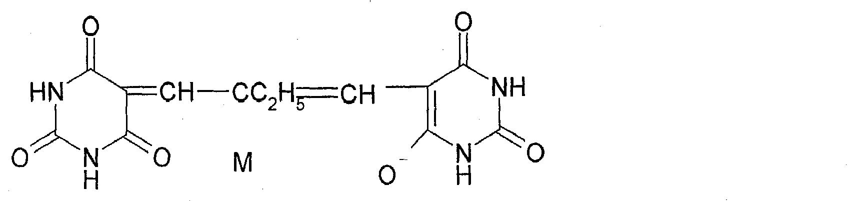 wherein M is as defined in claim 1.

11. The dye composition according to any of the preceding claims, such that it comprises from 0.0001% to 30% and preferably from 0.001% to 20% of methine direct dye(s) of formula I.

12. The dye composition according to any of the preceding claims, such that it also contains at least one conditioner, preferably chosen from cationic polymers, cations, silicones, chitosans and chitosan derivatives.

13. The dye composition according to Claim 12, containing at least one cationic polymer.

14. The dye composition according to one of the preceding claims containing at least one thickening polymer.

15. The dye composition according to one of the preceding claims, containing at least one surfactant chosen from the group consisting of anionic surfactants, amphoteric or zwitterionic surfactants, nonionic surfactants or cationic surfactants.

16. The dye composition according to one of the preceding claims, containing at least one oxidation dye precursor chosen from oxidation bases and couplers.

17. The dye composition according to Claim 16, comprising at least one oxidation base chosen from para-phenylenediamines, bis(phenyl)alkylenediamines, para-aminophenols, ortho-aminophenols and heterocyclic bases, and the addition salts thereof.

18. The dye composition according to Claim 16, comprising at least one coupler chosen from meta-phenylenediamines, meta-aminophenols, meta-diphenols, naphthalene-based couplers and heterocyclic couplers, and the addition salts thereof.

19. The dye composition according to Claim 18, wherein the coupler is chosen from 1,3-dihydroxybenzene, 1,3-dihydroxy-2-methylbenzene, 4-chloro-1,3-dihydroxybenzene, 2,4-diamino-1-(β-hydroxyethyloxy)benzene, 2-amino-4-(β-hydroxyethylamino)-1-methoxybenzene, 1,3-diaminobenzene, 1,3-bis(2,4-diaminophenoxy)propane, 3-ureidoaniline, 3-ureido-1-dimethylaminobenzene, sesamol, 1-β-hydroxyethylamino-3,4-methylenedioxybenzene, α-naphthol, 2-methyl-1-naphthol, 6-hydroxyindole, 4-hydroxyindole, 4-hydroxy-N-methylindole, 2-amino-3-hydroxypyridine, 6-hydroxybenzomorpholine, 3,5-diamino-2,6-dimethoxypyridine, 1-N-(β-hydroxyethyl)amino-3,4-methylenedioxybenzene and 2,6-bis(β-hydroxyethylamino)toluene, and the addition salts thereof.

20. The dye composition according to any of the preceding claims, comprising at least one additional direct dye other than the direct dye of formula I.

21. The dye composition according to any of the preceding claims, comprising at least one hydroxylated solvent.

22. The dye composition according to one of Claims 14 to 21 comprising at least one oxidizing agent chosen from hydrogen peroxide, urea peroxide, alkali metal bromates, persalts, peracids and oxidase enzymes.

23. A process for the oxidation dyeing of keratin fibres, comprising the steps of applying the dye composition according to one of Claims 1 to 22 to the keratin fibres.
